# EUROPÄISCHE PATENTANMELDUNG

(11) **EP 1 813 261 A2**
(43) Veröffentlichungstag der Anmeldung: **01.08.2007**
(21) Anmeldenummer: 06025547.8
(22) Anmeldetag: 11.12.2006
(51) Int. Cl.: A61K 8/64, A61Q 5/08

(54) **Blondierverfahren für keratinische Fasern**

(30) Priorität: 30.01.2006 DE 102006004354
(71) Anmelder: Henkel Kommanditgesellschaft auf Aktien, 40589 Düsseldorf (DE)
(72) Erfinder: Höffkes, Horst, 40595 Düsseldorf (DE); Eschen, Burkhard, 41468 Neuss (DE); Schulze Zur Wiesche, Erik, 20251 Hamburg (DE); Hollenberg, Detlef, 40699 Erkrath (DE)

(57) **Zusammenfassung**

Verfahren zur Blondierung keratinischer Fasern, insbesondere menschlicher Haare, bei dem in einem ersten Schritt eine Zubereitung A, enthaltend mindestens ein Oxidationsmittel, ausgewählt aus Wasserstoffperoxid und dessen Anlagerungsverbindungen an feste Träger, gegebenenfalls mit einer Zubereitung B, enthaltend mindestens einen Bleichkraftverstärker, gegebenenfalls mit einer Zubereitung C zu einer Anwendungszubereitung vermischt wird, diese in einem zweiten Schritt auf die keratinischen Fasern aufgetragen wird und anschließend nach einer Einwirkzeit t₁von den Fasern abgespült wird, wobei mindestens eine der Zubereitungen A, B oder C mindestens ein wasserlösliches Proteinhydrolysat oder dessen wasserlösliche Alkali- und/oder (gegebenenfalls substituierte) Ammonium- und/oder Erdalkalisalze enthält, so daß die Anwendungszubereitung mindestens ein wasserlösliches Proteinhydrolysat oder dessen wasserlösliche Alkali- oder Erdalkalisalze enthält, verbessern die innere Haarstruktur.

## Beschreibung

Gegenstand der vorliegenden Anmeldung sind Verfahren zur aufhellenden Farbveränderung keratinischer Fasern.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. Dabei können Dauerwell- und andere die Haarform verändernde Verfahren nahezu unabhängig vom Typ der zu behandelnden Haare eingesetzt werden. Dagegen sind Färbe- und insbesondere Blondierverfahren auf bestimmte Ausgangshaarfarben begrenzt. So eignen sich für aufhellende Verfahren, die so genannten Blondierverfahren, im Wesentlichen nur dunkelblonde oder hellere Haare. Die Grundlagen der Blondierverfahren sind dem Fachmann bekannt und in einschlägigen Monographien, z.B. von K. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, zusammenfassend beschrieben.

Zum Blondieren menschlicher Haare - insbesondere für die Strähnchenapplikation - werden üblicherweise feste oder pastenförmige Zubereitungen mit festen Oxidationsmitteln unmittelbar vor der Anwendung mit einer verdünnten Wasserstoffperoxid-lösung vermischt. Diese Mischung wird dann auf das Haar aufgebracht und nach einer bestimmten Einwirkzeit wieder ausgespült.

Um eine ausreichenden Blondierwirkung zu erzielen, sind derartige Mittel üblicherweise stark alkalisch eingestellt, der pH-Wert liegt dabei zwischen 9 und 10,5. Derart hohe pH-Werte sind erforderlich, um das Oxidationsmittel Wasserstoffperoxid zu aktivieren und um eine Öffnung der äußeren Schuppenschicht (Cuticula) zu gewährleisten und somit eine Penetration der aktiven Spezies (Wasserstoffperoxid) ins Haar zu ermöglichen. Als Alkalisierungsmittel wird üblicherweise Ammoniak eingesetzt, der allerdings den bekannten Nachteil des unangenehmen Geruchs aufweist.

Die für das Aufhellergebnis notwendige Öffnung der Cuticula kann in Einzelfällen von einer Schädigung der inneren Haarstruktur (Corneum) begleitet sein, insbesondere dann, wenn häufig, sehr lange oder mit sehr starken Blondiermitteln blondiert wird. Diese Schädigung rührt beispielsweise von der Zerstörung und/oder Eluierung von Melaninpartikeln in der Haarstruktur her.

Es besteht parallel zu dem Bedürfnis, die Haarfarbe zu verändern, üblicherweise der Wunsch, den Pflegezustand der Fasern nicht zu vernachlässigen. Daher werden herkömmlicher Weise die Fasern unmittelbar nach der farbverändernden Behandlung mit einem separat formulierten Pflegemittel behandelt.

In jüngster Zeit gab es stets Bestrebungen, die pflegenden Komponenten bereits mit in die Anwendungszubereitung einzuarbeiten. Dies führte aber häufig zu Problemen, da die eingesetzten Pflegestoffe unter den aggressiven Bedingungen (starke Oxidationsmittel, hoher pH-Wert) der aufhellenden Zubereitung nicht ausreichend stabil sind.

Der vorliegenden Erfindung lag die Aufgabe zugrunde, ein Verfahren zur Farbveränderung keratinischer Fasern, insbesondere menschlicher Haare, bereitzustellen, das eine Schädigung der Haare weitgehend vermeidet oder gar in der Lage ist, bereits erfolgte Schädigungen mindestens anteilsweise zu "reparieren", d.h. die Haarstruktur zu stärken.

Überraschenderweise wurde nunmehr gefunden, daß die Anwendung von wasserlöslichen Proteinhydrolysaten in aufhellenden Mitteln zur Verbesserung der inneren Haarstruktur führt.

Ein erster Gegenstand der vorliegenden Erfindung ist ein Verfahren zur Blondierung keratinischer Fasern, insbesondere menschlicher Haare, bei dem
- in einem ersten Schritt
   o eine Zubereitung A, enthaltend mindestens ein Oxidationsmittel, ausgewählt aus Wasserstoffperoxid und dessen Anlagerungsverbindungen an feste Träger,
   o gegebenenfalls mit einer Zubereitung B, enthaltend mindestens einen Bleichkraftverstärker,
   o gegebenenfalls mit einer Zubereitung C zu einer Anwendungszubereitung vermischt wird,
- diese in einem zweiten Schritt auf die keratinischen Fasern aufgetragen wird und
- anschließend nach einer Einwirkzeit t₁ von den Fasern abgespült wird,
wobei mindestens eine der Zubereitungen A, B oder C mindestens ein wasserlösliches Proteinhydrolysat oder dessen wasserlösliche Alkali- und/oder (gegebenenfalls substituierte) Ammonium- und/oder Erdalkalisalze enthält, so daß die Anwendungszubereitung mindestens ein wasserlösliches Proteinhydrolysat oder dessen wasserlösliche Alkali- oder Erdalkalisalze enthält.

Im erfindungsgemäßen Verfahren wird eine Anwendungsmischung auf die keratinischen Fasern, deren Farbe verändert werden soll, aufgebracht. Im einfachsten Fall enthält das Verkaufsprodukt bereits diese abgepackte Anwendungsmischung in Form der Zubereitung A, enthaltend mindestens ein Oxidationsmittel, ausgewählt aus Wasserstoffperoxid und dessen Anlagerungsverbindungen an feste Träger.

Um stärkere Farbveränderungen hervorzurufen, hat es sich bewährt, sogenannte Bleichkraftverstärker in die Anwendungsmischung einzuarbeiten. Da diese Substanzen in wäßriger Lösung zumeist nicht stabil sind und/oder die Lagerstabilität wasserstoffperoxidhaltiger Lösungen oder Dispersionen negativ beeinflussen, ist es bevorzugt, sie im Verkaufsprodukt getrennt von der Zubereitung A bereitzustellen und den Verbraucher die Anwendungszubereitung durch Vermischen der Zubereitung A mit dem Bleichkraftverstärker bzw. der Zubereitung, die den Bleichkraftverstärker enthält (Zubereitung B) direkt vor der Anwendung herstellen zu lassen. In einem solchen Fall kann das erfindungsgemäße Verfahren mit Verkaufsprodukten durchgeführt werden, die entweder in der Zubereitung A oder in der Zubereitung B oder in beiden Zubereitungen A und B mindestens ein wasserlösliches Proteinhydrolysat oder dessen wasserlösliche Alkali- und/oder (gegebenenfalls substituierte) Ammonium- und/oder Erdalkalisalze enthalten.

Wenn weitere Stoffe in die Anwendungsmischung inkorporiert werden sollen, die sowohl mit Wasserstoffperoxid als auch mit dem Bleichkraftverstärker inkompatibel sind, bietet es sich an, diese im Verkaufsprodukt in einer dritten Zubereitung C bereitzustellen und den Verbraucher die Anwendungszubereitung durch Vermischen der Zubereitung A mit dem Bleichkraftverstärker bzw. der Zubereitung, die den Bleichkraftverstärker enthält (Zubereitung B) und der Zubereitung, die den weiteren Stoff enthält (Zubereitung C) direkt vor der Anwendung herstellen zu lassen. In einem solchen Fall kann das erfindungsgemäße Verfahren mit Verkaufsprodukten durchgeführt werden, die entweder in der Zubereitung A oder in der Zubereitung B oder in der Zubereitung C oder in beiden Zubereitungen A und B oder in beiden Zubereitungen A und C oder in beiden Zubereitungen B und C oder in allen drei Zubereitungen A, B und C mindestens ein wasserlösliches Proteinhydrolysat oder dessen wasserlösliche Alkali- und/oder (gegebenenfalls substituierte) Ammonium- und/oder Erdalkalisalze enthalten.

Selbstverständlich kann der vorstehend beschriebene Fall auch ohne zusätzlichen Bleichkraftverstärker realisiert werden. In diesem Fall ist die Zubereitung C eine zweite Zubereitung im Verkaufsprodukt, und der Verbraucher stellt die Anwendungszubereitung durch Vermischen der Zubereitung A mit der Zubereitung, die den weiteren Stoff enthält (Zubereitung C) direkt vor der Anwendung her. In einem solchen Fall kann das erfindungsgemäße Verfahren mit Verkaufsprodukten durchgeführt werden, die entweder in der Zubereitung A oder in der Zubereitung C oder in beiden Zubereitungen A und C mindestens ein wasserlösliches Proteinhydrolysat oder dessen wasserlösliche Alkali- und/oder (gegebenenfalls substituierte) Ammonium- und/oder Erdalkalisalze enthalten.

Enthält eine Verkaufsverpackung zusätzlich zur Zubereitung A eine Zubereitung B und/oder eine Zubereitung C, so wird die Anwendungszubereitung durch Vermischen hergestellt und auf die keratinischen Fasern aufgetragen (Schritt 1 des erfindungsgemäßen Verfahrens) und nach einer Einwirkzeit t₁ von dort wieder abgespült. Die Einwirkzeit t₁ beträgt vorzugsweise eine bis 60 Minuten, besonders bevorzugt 5 bis 50 Minuten und insbesondere 10 bis 45 Minuten.

In besonders bevorzugten erfindungsgemäßen Verfahren erfolgt im Anschluß an das Ausspülen der Anwendungszubereitung eine Nachspülung mit einer weiteren Zubereitung D. Solche bevorzugten erfindungsgemäßen Verfahren sind dadurch gekennzeichnet, daß
- in einem dritten Schritt eine wäßrige Zubereitung D eines oder mehrerer wasserlöslicher Salze von Calcium und/oder Aluminium und/oder Zink und/oder der Seltenerdmetalle auf die keratinischen Fasern aufgetragen wird und diese
- anschließend nach einer Einwirkzeit t₂ von den Fasern abgespült wird.

Die Einwirkzeit t₂ beträgt vorzugsweise 10 Sekunden bis 60 Minuten, besonders bevorzugt 30 Sekunden bis 50 Minuten und insbesondere 1 bis 45 Minuten.

Bei diesem bevorzugten erfindungsgemäßen Verfahren wird die wäßrige Zubereitung D als Nachbehandlungsmittel eingesetzt und dient einer weiteren Fixierung der Proteinhydrolysate oder deren Anionen durch die Bildung unlöslicher Salze. Die wäßrige Zubereitung D kann aber auch als Vorbehandlungsmittel eingesetzt werden, woraus ein erfindungsgemäßes bei Verfahren zur Blondierung keratinischer Fasern, insbesondere menschlicher Haare, resultiert, bei dem
- in einem ersten Schritt eine wäßrige Zubereitung D eines oder mehrerer wasserlöslicher Salze von Calcium und/oder Aluminium und/oder Zink und/oder der Seltenerdmetalle auf die keratinischen Fasern aufgetragen wird und diese
- anschließend nach einer Einwirkzeit t₂ von den Fasern abgespült wird, wonach
- in einem zweiten Schritt
   o eine Zubereitung A, enthaltend mindestens ein Oxidationsmittel, ausgewählt aus Wasserstoffperoxid und dessen Anlagerungsverbindungen an feste Träger,
   o gegebenenfalls mit einer Zubereitung B, enthaltend mindestens einen Bleichkraftverstärker,
   o gegebenenfalls mit einer Zubereitung C zu einer Anwendungszubereitung vermischt wird,
- diese in einem dritten Schritt auf die keratinischen Fasern aufgetragen wird und
- anschließend nach einer Einwirkzeit t₁ von den Fasern abgespült wird,
wobei mindestens eine der Zubereitungen A, B oder C mindestens ein wasserlösliches Proteinhydrolysat oder dessen wasserlösliche Alkali- und/oder (gegebenenfalls substituierte) Ammonium- und/oder Erdalkalisalze enthält, so daß die Anwendungszubereitung mindestens ein wasserlösliches Proteinhydrolysat oder dessen wasserlösliche Alkali- oder Erdalkalisalze enthält.

Die im erfindungsgemäßen Verfahren eingesetzten Mittel zur aufhellenden Farbveränderung keratinischer Fasern enthalten (in der Zubereitung A) mindestens ein Oxidationsmittel, ausgewählt aus Wasserstoffperoxid und dessen Anlagerungsverbindungen an feste Träger.

Als erfindungsgemäßes Oxidationsmittel wird bevorzugt Wasserstoffperoxid selbst verwendet. Das Wasserstoffperoxid wird als Lösung oder in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidon.n H₂O₂ (n ist eine positive ganze Zahl größer 0), Harnstoffperoxid und Melaminperoxid, eingesetzt.

Erfindungsgemäß ganz besonders bevorzugt sind wässrige WasserstoffperoxidLösungen. Die Konzentration einer Wasserstoffperoxid-Lösung wird einerseits von den gesetzlichen Vorgaben und andererseits von dem gewünschten Effekt bestimmt; vorzugsweise werden 6- bis 12-prozentige Lösungen in Wasser verwendet.

Gegebenenfalls wird im erfindungsgemäßen Verfahren auch eine Zubereitung B eingesetzt, die Bleichkraftverstärker enthält. Vorzugsweise ist der Bleichkraftverstärker ausgewählt aus Ammoniumpersulfat, Alkalimetallpersulfaten, Ammoniumperoxomonosulfat, Alkalimetallhydrogenperoxomonosulfaten, Alkalimetallperoxidiphosphaten und Erdalkalimetallperoxiden. Besonders bevorzugte Bleichkraftverstärker sind Ammoniumperoxidisulfat, Kaliumperoxidisulfat, Natriumperoxidisulfat, Kaliumhydrogenperoxomonosulfat, Kaliumperoxidiphosphat, Magnesiumperoxid und Bariumperoxid.

Weiterhin hat es sich bei den Arbeiten zur vorliegenden Erfindung als besonders bevorzugt erwiesen, wenn die erfindungsgemäßen Mittel mindestens zwei verschiedene Peroxidisulfate enthalten.

Die erfindungsgemäßen wasserfreien Zusammensetzungen anstelle und/oder zusätzlich zu den festen Peroxoverbindungen einen weiteren Bleichkraftverstärker enthalten.

Als Bleichverstärker können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Zur weiteren Steigerung der Aufhellleistung können der erfindungsgemäßen Zusammensetzung zusätzlich als Bleichverstärker mindestens eine gegebenenfalls hydratisierte SiO₂-Verbindung zugesetzt. Obwohl bereits geringe Mengen der gegebenenfalls hydratisierten SiO₂-Verbindungen die Aufhellleistung erhöhen, kann es erfindungsgemäß bevorzugt sein, die gegebenenfalls hydratisierten SiO₂-Verbindunge in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der SiO₂-Verbindungen (ohne deren Wasseranteil) in den Mitteln wieder.

Hinsichtlich der gegebenenfalls hydratisierten SiO₂-Verbindungen unterliegt die vorliegende Erfindung prinzipiell keinen Beschränkungen. Bevorzugt sind Kieselsäuren, deren Oligomeren und Polymeren sowie deren Salze. Bevorzugte Salze sind die Alkalisalze, insbesondere die Kalium und Natriumsalze. Die Natriumsalze sind ganz besonders bevorzugt.

Die gegebenenfalls hydratisierten SiO₂-Verbindungen können in verschiedenen Formen vorliegen. Erfindungsgemäß bevorzugt werden die SiO₂-Verbindungen in Form von Kieselgelen (Silicagel) oder besonders bevorzugt als Wasserglas eingesetzt. Diese SiO₂-Verbindungen können teilweise in wäßriger Lösung vorliegen.

Erfindungsgemäß ganz besonders bevorzugt sind Wassergläser, die aus einem Silikat der Formel (SiO₂)ₙ(Na₂O)ₘ(K₂O)ₚ gebildet werden, wobei n steht für eine positive rationale Zahl und m und p stehen unabhängig voneinander für eine positive rationale Zahl oder für 0, mit den Maßgaben, daß mindestens einer der Parameter m oder p von 0 verschieden ist und das Verhältnis zwischen n und der Summe aus m und p zwischen 1:4 und 4:1 liegt.

Neben den durch die Summenformel beschriebenen Komponenten können die Wassergläser in geringen Mengen noch weitere Zusatzstoffe, wie beispielsweise Phosphate oder Magnesiumsalze, enthalten.

Erfindungsgemäß besonders bevorzugte Wassergläser werden unter anderem von der Firma Henkel unter den Bezeichnungen Ferrosil^{®} 119, Natronwasserglas 40/42, Portil^{®} A, Portil^{®} AW und Portil^{®} W und von der Firma Akzo unter der Bezeichnung Britesil^{®} C20 vertrieben.

Als Bleichverstärker können erfindungsgemäß bevorzugt Carbonatsalze, bzw. Hydrogencarbonatsalze eingesetzt werden. Diese sind bevorzugt ausgewählt aus der Gruppe der Ammonium-, Alkali- (insbesondere Natrium- und Kalium-), sowie Erdalkali-(insbesondere Calcium-), -carbonatsalze bzw. -hydrogencarbonatsalze. Besonders bevorzugte Carbonat-, bzw. Hydrogencarbonatsalze sind Ammoniumhydrogencarbonat, Ammoniumcarbonat, Natriumhydrogencarbonat, Dinatriumcarbonat, Kaliumhydrogencarbonat, Dikaliumcarbonat und Calciumcarbonat. Diese besonders bevorzugten Salze können allein oder in deren Mischungen von mindestens zwei Vertretern als Bleichverstärker verwendet werden.

Als Bleichverstärker vom Typ der Monoalkylcarbonate und deren Derivate werden bevorzugt mindestens ein Kohlensäuremonoester und/oder mindestens ein Kohlensäuremonoamid in dem erfindungsgemäßen Verfahren verwendet. Bevorzugt verwendbare Kohlensäuremonoester sind die Kohlensäuremonoester der Formel (1),

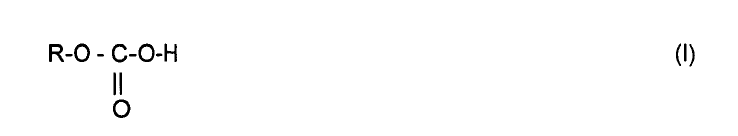

in der R für einen gesättigten oder ungesättigten, geradkettigen, verzweigten, oder cyclischen, substituierten oder unsubstituierten Kohlenwasserstoffrest, oder eine substituierte oder unsubstituierte Arylgruppe bzw. einen substituierten oder unsubstituierten Heterocyclus steht.

In Formel (I) steht R vorzugsweise für einen substituierten oder unsubstituierten, geradkettigen oder verzweigten Alkyl-, Alkenyl- oder Alkinylrest, wobei als Substituenten bevorzugt Hydroxy-, Amino-, Nitro-, Sulfonsäuregruppen oder Halogene in Frage kommen. Weitere bevorzugte Reste R sind Phenyl- und Benzylreste sowie weiter substituierte Vertreter. Besonders bevorzugt steht R für eine C₁₋₆-Alkylgruppe. Beispiele für erfindungsgemäße C₁-C₆-Alkylgruppen sind die Gruppen Methyl, Ethyl, n-Propyl, Isopropyl, n-Butyl, sec-Butyl, iso-Butyl, tert-Butyl, Pentyl, iso-Pentyl und Hexyl.

Erfindungsgemäß besonders bevorzugt verwendete Zusammensetzungen sind dadurch gekennzeichnet, daß der Rest R in Formel (I) ausgewählt ist aus Methyl-, Ethyl-, n-Propyl-, iso-Propyl-, n-Butyl-, iso-Butyl-, *tert*-Butyl- sowie Hydroxymethyl- und Hydroxyethyl-Resten.

Alternativ zum Kohlensäuremonoester oder in Verbindung mit ihm können in den wasserfreien Zusammensetzungen Kohlensäuremonoamide als Bleichverstärker eingesetzt werden. Hier ist es erfindungsgemäß bevorzugt, mindestens ein Kohlensäuremonoamid der Formel (II) zu verwenden, 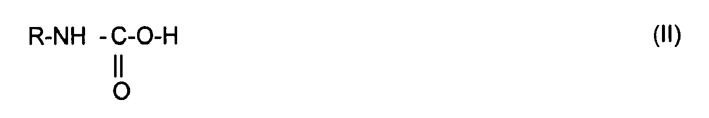 in der R für einen gesättigten oder ungesättigten, geradkettigen, verzweigten, oder cyclischen, substituierten oder unsubstituierten Kohlenwasserstoffrest, oder eine substituierte oder unsubstituierte Arylgruppe bzw. einen substituierten oder unsubstituierten Heterocyclus steht.

In Formel (II) steht R vorzugsweise für einen substituierten oder unsubstituierten, geradkettigen oder verzweigten Alkyl-, Alkenyl- oder Alkinylrest, wobei als Substituenten bevorzugt Hydroxy-, Amino-, Nitro-, Sulfonsäuregruppen oder Halogene in Frage kommen. Weitere Bevorzugte Reste R sind Phenyl- und Benzylreste sowie weiter substituierte Vertreter. Besonders bevorzugt steht R für eine C₁₋₆-Alkylgruppe. Beispiele für erfindungsgemäße C₁-C₆-Alkylgruppen sind die Gruppen Methyl, Ethyl, n-Propyl, Isopropyl, n-Butyl, sec-Butyl, iso-Butyl, tert-Butyl, Pentyl, iso-Pentyl und Hexyl.

Erfindungsgemäß besonders bevorzugte Bleichverstärker der Formel (II) sind dadurch gekennzeichnet, daß der Rest R in Formel (II) ausgewählt ist aus Methyl-, Ethyl-, n-Propyl-, iso-Propyl-, n-Butyl-, iso-Butyl-, *tert*-Butyl- sowie Hydroxymethyl- und Hydroxyethyl-Resten.

Das acide H-Atom des Kohlensäuremonoesters bzw. -monoamids kann auch in neutralisierter Form vorliegen, d.h. es können erfindungsgemäß auch Salze von Kohlensäuremonoestern bzw. Kohlensäuremonoamiden eingesetzt werden. Hier sind erfindungsgemäß Kohlensäuremonoester bzw. das Kohlensäuremonoamide bevorzugt, die in ganz oder teilweise neutralisierter Form, vorzugsweise in Form des Alkalimetall-, Ammonium-, Erdalkalimetall- oder Aluminiumsalzes und insbesondere in Form seines Natriumsalzes, vorliegen.

Als Bleichverstärker vom Typ der Silylcarbonate und deren Derivate werden bevorzugt mindestens ein Silylcarbonat und/oder mindestens ein Silylcarbamat in die erfindungsgemäßen Zusammensetzungen eingearbeitet. Bevorzugt werden Silylcarbonate gemäß Formel (III) eingesetzt,

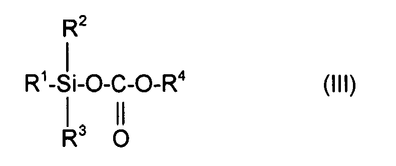

in der die Reste R¹, R² und R³ unabhängig voneinander für ein Wasserstoffatom, einen gesättigten oder ungesättigten, geradkettigen, verzweigten, oder cyclischen, substituierten oder unsubstituierten Kohlenwasserstoffrest oder für eine Trialkylsilylgruppe, vorzugsweise eine Trimethylsilylgruppe oder für eine substituierte oder unsubstituierte Arylgruppe bzw. einen substituierten oder unsubstituierten Heterocyclus oder für ein Halogen, eine substituierte oder unsubstituierte Hydroxy-, Oxo- , Aminogruppen stehen und der Rest R⁴ für eine chemische Bindung zum Si-Atom oder zu einem der Reste R¹, R² oder R³, ein Wasserstoffatom, einen gesättigten oder ungesättigten, geradkettigen, verzweigten, oder cyclischen, substituierten oder unsubstituierten Kohlenwasserstoffrest oder für eine substituierte oder unsubstituierte Silyl- oder Aluminylgruppe oder für eine substituierte oder unsubstituierte Arylgruppe bzw. einen substituierten oder unsubstituierten Heterocyclus steht.

Bevorzugte Reste R¹, R² und R³ in der oben genannten Formel (III) sind substituierte oder unsubstituierte, geradkettige oder verzweigte Alkylreste. Unter diesen sind die bevorzugte erfindungsgemäße wasserfreie Zusammensetzungen dadurch gekennzeichnet sind, daß die Reste R¹, R² und R³ in Formel (III) ausgewählt sind aus Methyl-, Ethyl-, n-Propyl-, iso-Propyl-, n-Butyl-, iso-Butyl-, *tert*-Butyl- sowie Hydroxymethyl- und Hydroxyethyl-Resten.

Bevorzugte Reste R⁴ in der oben genannten Formel (III) sind Wasserstoff, substituierte oder unsubstituierte, geradkettige oder verzweigte Alkylreste sowie Trialkylsilylreste. Unter diesen sind Wasserstoff, Methyl-, Ethyl-, tert.-Butyl- und Trimethylsilylreste bevorzugt.

Als Bleichverstärker kann mindestens ein Silylcarbamat der Formel (IV) in der erfindungsgemäßen wasserfreien Zusammensetzung enthalten sein, 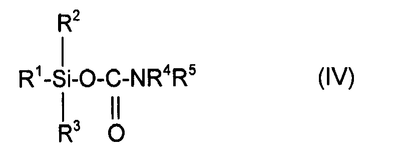 wobei die Reste R¹, R² und R³ unabhängig voneinander für ein Wasserstoffatom, einen gesättigten oder ungesättigten, geradkettigen, verzweigten, oder cyclischen, substituierten oder unsubstituierten Kohlenwasserstoffrest oder für eine Trialkylsilylgruppe, vorzugsweise eine Trimethylsilylgruppe oder für eine substituierte oder unsubstituierte Arylgruppe bzw. einen substituierten oder unsubstituierten Heterocyclus oder für ein Halogen, eine substituierte oder unsubstituierte Hydroxy-, Oxo- , Aminogruppen stehen und die Rest R⁴ und R⁵ unabhängig voneinander für eine chemische Bindung zum Si-Atom oder zu einem der Reste R¹, R² oder R³, ein Wasserstoffatom, einen gesättigten oder ungesättigten, geradkettigen, verzweigten, oder cyclischen, substituierten oder unsubstituierten Kohlenwasserstoffrest oder für eine substituierte oder unsubstituierte Silyl- oder Aluminylgruppe oder für eine substituierte oder unsubstituierte Arylgruppe bzw. einen substituierten oder unsubstituierten Heterocyclus stehen.

Bevorzugte Reste R¹, R² und R³ in der oben genannten Formel (IV) sind substituierte oder unsubstituierte, geradkettige oder verzweigte Alkylreste. Unter diesen sind die Alkylreste mit 1 bis 5 Kohlenstoffatomen sowie die Hydroxyalkylreste bevorzugt, so daß bevorzugt verwendete Zusammensetzungen dadurch gekennzeichnet sind, daß die Reste R¹, R² und R³ in Formel (IV) ausgewählt sind aus Methyl-, Ethyl-, n-Propyl-, isoPropyl-, n-Butyl-, iso-Butyl-, *tert*-Butyl- sowie Hydroxymethyl- und Hydroxyethyl-Resten.

Bevorzugte Reste R⁴ und R⁵ in der oben genannten Formel (IV) sind Wasserstoff, substituierte oder unsubstituierte, geradkettige oder verzweigte Alkylreste sowie Trialkylsilylreste. Unter diesen sind Wasserstoff, Methyl-, Ethyl-, tert.-Butyl- und Trimethylsilylreste bevorzugt.

Als weitere zusätzliche Bleichverstärker können in den erfindungsgemäßen Zusammensetzungen bevorzugt mindestens eine Verbindung ausgewählt aus Essigsäure, Milchsäure, Weinsäure, Zitronensäure, Salicylsäure und ortho-Phthalsäure, enthalten sein.

Im Rahmen einer ersten bevorzugten Ausführungsform wird die Anwendungsmischung für das erfindungsgemäße Verfahren unmittelbar vor der Anwendung durch Mischung einer Zubereitung (A), enthaltend mindestens ein Oxidationsmittel, ausgewählt aus Wasserstoffperoxid und dessen Anlagerungsverbindungen an feste Träger, gegebenenfalls einer Zubereitung (B) enthaltend mindestens einen Bleichkraftverstärker sowie gegebenenfalls einer Zubereitung (C hergestellt, wobei die Anwendungsmischung mindestens ein wasserlösliches Proteinhydrolysat oder dessen wasserlösliche Alkali- und/oder (gegebenenfalls substituierte) Ammonium- und/oder Erdalkalisalze enthält.

Bevorzugte erfindungsgemäße Verfahren sind dadurch gekennzeichnet, daß die Zubereitung A wässrig ist.

Die Zubereitung (B), enthaltend mindestens einen Bleichkraftverstärker, ist vorzugsweise pulverförmig formuliert, wobei bevorzugte erfindungsgemäße Verfahren dadurch gekennzeichnet sind, daß die Zubereitung B wasserfrei formuliert ist, und wobei bevorzugte Zubereitungen B pulverförmig sind.

Obwohl prinzipiell keine Einschränkungen hinsichtlich der Formulierung der Zubereitung (B) bestehen, hat es sich erfindungsgemäß als bevorzugt erwiesen, wenn die Zubereitung (B) wasserfrei formuliert ist.

Wasserfrei im Sinne der vorliegenden Erfindung bedeutet einen Wassergehalt bezogen auf die Zubereitung (B) von weniger als 5 Gew.-%, insbesondere von weniger als 2 Gew.-%.

Zubereitungen, die weniger als 0,1 Gew.-% Wasser, enthalten können erfindungsgemäß ganz besonders bevorzugt sein.

Die Zubereitung (B) ist vorzugsweise als Pulver oder als wasserfreie Paste formuliert.

Im Falle der Formulierung als wasserfreie Paste hat es sich als besonders bevorzugt erwiesen, wenn die Zubereitung (B) mindestens einen nicht hydroxylierten Fettsäureester mit einem Schmelzpunkt von höchstens 50°C, insbesondere von höchstens 30°C, und/oder mindestens eine C₁₀- bis C₃₀-Fettsäure mit mindestens einer zusätzlichen Hydroxygruppe und/oder ein Derivat davon, enthält.

Ester von nicht hydroxylierten C₆- bis C₃₀-Alkylmonocarbonäuren mit C₂- bis C₃₀-Monoalkoholen eignen sich erfindungsgemäß bevorzugt als Fettsäureester. Bevorzugt sind die Monoester der Fettsäuren mit Monoalkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Estern sind lsopropylalkohol, Capronalkohol, Caprylalkohol, 2-Ethylhexylalkohol, Caprinalkohol, Laurylalkohol, lsotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Kokosfettalkohol-caprinat/-caprylat (Cetiol^{®} LC), n-Butylstearat, Oleylerucat (Cetiol^{®} J 600), Isopropylpalmitat (Rilanit^{®} IPP), Oleyloleat (Cetiol^{®}), Laurinsäurehexylester (Cetiol^{®} A), Myristylmyristat (Cetiol^{®} MM), Cetearylisononanoat (Cetiol^{®} SN), Ölsäuredecylester (Cetiol^{®} V).

Die erfindungsgemäßen wasserfreien Zusammensetzungen enthalten die nicht hydroxylierten Fettsäureester (A1) bevorzugt in einer Menge von 2 bis 60 Gew.-%, besonders bevorzugt von 5 bis 30 Gew.-%, jeweils bezogen auf das Gewicht der wasserfreien Zusammensetzung.

Die C₁₀- bis C₃₀-Fettsäure mit mindestens einer zusätzlichen Hydroxygruppe (A2) bzw. deren Derivate können sich von einer gesättigten oder ungesättigten Fettsäure ableiten die bevorzugt ein bis drei zusätzliche Hydroxygruppen tragen. Bevorzugt handelt es sich erfindungsgemäß um gesättigte Fettsäuren bzw. Derivate davon.

Wiederum bevorzugt eignen sich als Fettsäure gemäß (A2) die Monohydroxy-C₁₀- bis C₃₀-Carbonsäuren und deren Derivate. Als Derivate eignen sich insbesondere die Ester mit mindestens einem Vertreter der Fettalkohole, insbesondere derjenigen Fettalkohole, die unter den im Zusammenhang mit den Estern (A1) genannten Fettalkoholanteilen erwähnt werden.
Dabei wird die C₁₀- bis C₃₀-Fettsäure mit mindestens einer zusätzlichen Hydroxygruppe insbesondere ausgewählt aus Verbindungen der Formel (V) und/oder deren physiologisch verträglichen Salzen, 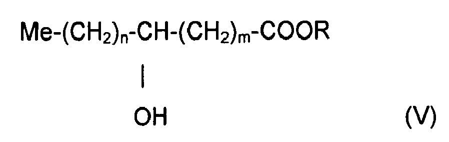 worin
R steht für ein Wasserstoffatom oder eine C₈- bis C₂₂-Alkylgruppe,
m eine ganze Zahl von 0 bis 27 und
n eine ganze Zahl von 0 bis 27 bedeuten, sowie
die Summe m + n eine ganze Zahl von 7 bis 27 beträgt.

Dabei ist es erneut bevorzugt, wenn die C₁₀- bis C₃₀-Fettsäure mit mindestens einer zusätzlichen Hydroxygruppe ausgewählt wird aus solchen Verbindungen der Formel (V) bzw. deren physiologisch verträglichen Salzen, die durch die Parameter R bedeutet ein Wasserstoffatom oder eine C₈- bis C₂₂-Alkylgruppe,
m eine ganze Zahl von 2 bis 27,
n eine ganze Zahl von 0 bis 27, sowie
die Summe m + n eine ganze Zahl von 7 bis 27
definiert sind.

Solche C₁₀- bis C₃₀-Fettsäuren mit mindestens einer zusätzlichen Hydroxygruppe bzw. deren Derivate sind beispielsweise 12-Hydroxystearinsäure, 3-Hydroxypalmitinsäure, 12-Hydroxylaurinsäure, 16-Hydroxypalmitinsäure und 17-Hydroxypalmitinsäure bzw. deren physiologisch verträglichen Salze.

Ganz besonders bevorzugt ist die C₁₀- bis C₃₀-Fettsäure mit mindestens einer zusätzlichen Hydroxygruppe bzw. deren Derivate 12-Hydroxystearinsäure und/oder eines ihrer Derivate und/oder eines ihrer physiologisch verträglichen Salze. 12-Hydroxystearinsäurederivate sind als Esterderivat insbesondere in hydriertem Rizinusöl enthalten und als solches beispielsweise als Handelsprodukte Cutina^{®} HR (INCI-Bezeichnung: Hydrogenated Castor Oil; Fa. Cognis), Rilanit^{®} HT und Rilanit^{®} GTH (Fa. Cognis) erhältlich.

Die erfindungsgemäßen wasserfreien Zusammensetzungen enthalten die C₁₀- bis C₃₀-Fettsäuren mit mindestens einer zusätzlichen Hydroxygruppe bzw. deren Derivate bevorzugt in einer Menge von 0.1 bis 5 Gew.-%, besonders bevorzugt von 0.5 bis 3 Gew.-%, jeweils bezogen auf das Gewicht der wasserfreien Zusammensetzung.

Die erfindungsgemäßen wasserfreien Zusammensetzungen enthalten bevorzugt im Gewichtsverhältnisbereich der nicht hydroxylierten Fettsäureestern (A1) zu den C₁₀- bis C₃₀-Fettsäuren mit mindestens einer zusätzlichen Hydroxygruppe bzw. deren Derivaten (A2) von 10 zu 1 bis 60 zu 1.

Weiter verbessert werden die wasserfreien Zusammensetzungen, durch einen Zusatz mindestens eines Vertreters der Gruppe, bestehend aus Partialglyceriden, Fettalkoholen und Guerbetalkoholen, insbesondere mindestens eines Vertreters der der Gruppe bestehend aus Fettalkoholen und Guerbetalkoholen. Der besagte Zusatz ist bevorzugt in einer Menge von 0.1 bis 20 Gew.-%, besonders bevorzugt von 0.5 bis 10 Gew.-%, jeweils bezogen auf das Gewicht der wasserfreien Zusammensetzung, enthalten.

Partialglyceride, also Monoglyceride, Diglyceride und deren technische Gemische können herstellungs-bedingt noch geringe Mengen Triglyceride enthalten. Die Partialglyceride folgen vorzugsweise der Formel (VI), 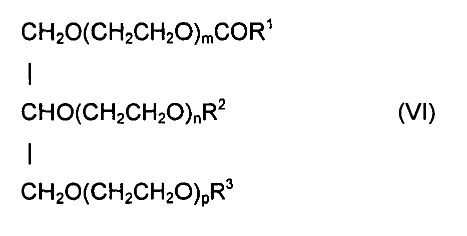 in der COR¹ für einen linearen oder verzweigten, gesättigten und/oder ungesättigten Acylrest mit 6 bis 22, vorzugsweise 12 bis 18 Kohlenstoffatomen, R² und R³ unabhängig voneinander für COR¹ oder OH und die Summe (m+n+p) für 0 oder Zahlen von 1 bis 100, vorzugsweise 5 bis 25 steht, mit der Maßgabe, daß mindestens einer der beiden Reste R² und R³ OH bedeutet.

Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden technische Laurinsäureglyceride, Palmitinsäureglyceride, Stearinsäureglyceride, Isostearinsäureglyceride, Ölsäureglyceride, Behensäureglyceride und/oder Erucasäureglyceride eingesetzt, welche einen Monoglyceridanteil im Bereich von 50 bis 95, vorzugsweise 60 bis 90 Gew.-% aufweisen.

Bevorzugte Fettalkohole sind primäre aliphatische Alkohole der Formel (VII),

R¹OH (VII)

in der R¹ für einen aliphatischen, linearen oder verzweigten Kohlenwasserstoffrest mit 8 bis 22 Kohlenstoffatomen, der gesättigt ist oder bis zu 3 Doppelbindungen enthalten kann, steht.

Typische Beispiele sind 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen.

Bevorzugt sind Fettalkoholmischungen, die Fettalkohole mit 12 bis 18 Kohlenstoffatomen enthalten, wie beispielsweise solche Mischungen, die aus Kokos-, Palm-, Palmkernöl oder Talgfett, insbesondere aus Kokosöl oder Talgfett, gewonnen werden.

Unter Guerbetalkoholen sind Alkohole zu verstehen, die durch alkalische Kondensation von Alkoholen zu höhermolekularen, verzweigten Iso-Alkoholen hergestellt werden. Diese Umsetzung wurde erstmals von Guerbet 1899 veröffentlicht. Machemer stellte 1952 wesentliche Schritte der Reaktion dar (Angewandte Chemie 64 (1952) 213 - 20): Neben der Dehydrierung zum Keton, bei der Wasserstoff abgespalten wird, und der Aldolkondensation ist die Crotonisierung, bei der Wasser abgespalten wird, ein wichtiger Schritt im Reaktionsablauf. Stand der Technik ist eine Reaktionsführung bei Normaldruck und einer Reaktionstemperatur von 240 bis 260°C. Die so erhaltenen verzweigten Alkohole werden als Guerbetalkohole bezeichnet. Aus dem Stand der Technik sind inzwischen eine Vielzahl weiterer Verfahren bekannt, gemäß derer man Guerbetalkohole erhalten kann.

Besonders bevorzugt besitzen die Fett- bzw. Guerbetalkohole der erfindungsgemäßen wässrigen Zusammensetzung einen Schmelzpunkt von höchstens 50°C, insbesondere höchstens 30°C.

Die Zubereitung (C) stellt vorzugsweise eine typische wässrige Cremegrundlage dar.

Ein Beispiel für eine derartige Cremegrundlage stellt die folgende Rahmenrezeptur dar:

| | |
|---|---|
| Fettalkohol / -säure | 13-17 Gew. % |
| Tensid (z.B. Fettalkoholethersulfat, Alkylpolyglycosid) | 10 - 15 Gew. % |
| Emulgator (z.B. Fettalkoholethoxylate) | 0 - 1,2 Gew. % |
| Reduktionsmittel (z.B. Natriumsulfit; Ascorbinsäure) | 0,2 - 2 Gew. % |
| Alkalisierungsmittel (z.B. Ammoniak, 2-Aminoethanol) | 6-10 Gew. % |
| Komplexbildner und Wasserglas | ca. 1 Gew. % |
| Parfüm | 0,2 - 0,5 Gew. % |
| Wasser | ad 100 |

Das erfindungsgemäße Verfahren ist dadurch gekennzeichnet, daß die Anwendungsmischung mindestens ein wasserlösliches Proteinhydrolysat oder dessen wasserlösliche Alkali- und/oder (gegebenenfalls substituierte) Ammonium- und/oder Erdalkalisalze enthält.

Als Proteine, aus denen die Proteinhydrolysate gewonnen werden können, kommen im Rahmen der vorliegenden Erfindung grundsätzlich alle Proteine unabhängig von ihrem Ursprung oder ihrer Herstellung in Betracht. Beispiele für Proteine tierischen Ursprungs sind Keratin, Elastin, Kollagen, Fibroin, Albumin, Casein, Molkeprotein, Plazentaprotein. Erfindungsgemäß bevorzugt aus diesen sind Kollagen, Keratin, Casein, Molkeprotein, Proteine pflanzlichen Ursprungs wie beispielsweise Weizen- und Weizenkeimprotein, Reisprotein, Sojaprotein, Haferprotein, Erbsenprotein, Kartoffelprotein, Mandelprotein und Hefeprotein können erfindungsgemäß ebenfalls bevorzugt sein.

Unter Proteinhydrolysaten sind im Rahmen der vorliegenden Erfindung Abbauprodukte von Proteinen wie beispielsweise Kollagen, Elastin, Casein, Keratin, Mandel-, Kartoffel-, Weizen-, Reis- und Sojaprotein zu verstehen, die durch saure, alkalische und / oder enzymatische Hydrolyse der Proteine selbst oder ihrer Abbauprodukte wie beispielsweise Gelatine erhalten werden. Für den enzymatischen Abbau sind alle hydrolytisch wirkenden Enzyme geeignet, wie z. B. alkalische Proteasen.

Erfindungsgemäß bevorzugte Verfahren sind dadurch gekennzeichnet, daß mindestens eine der Zubereitungen A und/oder B und/oder C mindestens ein wasserlösliches Proteinhydrolysat mit Molmassen von 400 bis 10.000 Dalton, vorzugsweise von 1000 bis 5000 Dalton oder dessen wasserlösliche Alkali- oder Erdalkalisalze enthält.

Besonders bevorzugt sind erfindungsgemäße Verfahren, bei denen sämtliche in den Zubereitungen A und/oder B und/oder C enthaltenen wasserlöslichen Proteinhydrolysate und/oder deren wasserlösliche Alkali- und/oder (gegebenenfalls substituierte) Ammonium- und/oder Erdalkalisalze Molmassen von 400 bis 10.000 Dalton, vorzugsweise von 1000 bis 5000 Dalton aufweisen.

Als Alkalisalze haben sich insbesondere die Natrium- und Kaliumsalze bewährt, bei den Erdalkalisalzen sind oft lediglich die Magnesiumsalze wasserlöslich. Ammoniumsalze (NH₄⁺) und Alkylammoniumsalze, also Monoykylammonium-, Dialkylammonium-, Trialkylammonium- und Tetraalkylammoniumsalze sind ebenfalls geeignet, wenn sie ausreichend wasserlöslich sind, wobei unter wasserlöslich im Rahmen der vorliegenden Erfindung eine Löslichkeit von mindestens 1 Gramm pro Liter destilliertem Wasser bei 20°C verstanden wird.

Ein bevorzugt im erfindungsgemäßen Verfahren einzusetzendes Proteinhydrolysat wird aus Casein gewonnen, so daß bevorzugte erfindungsgemäße Verfahren dadurch gekennzeichnet sind, daß das bzw. die wasserlösliche(n) Proteinhydrolysat(e) oder dessen bzw deren wasserlösliche(s) Alkali- und/oder (gegebenenfalls substituierte) Ammonium- und/oder Erdalkalisalz(e) ausgewählt ist aus Caseinhydrolysaten.

Unabhängig von der Art des Proteins, aus dem die niedermolekularen Hydrolysate gewonnen werden, sind Hydrolysate bevorzugt, die bestimmte Aminosäuren in bestimmten Mengen enthalten. Diese bevorzugten Hydrolysate werden nachstehend beschrieben:

So sind insbesondere erfindungsgemäße Verfahren bevorzugt, bei denen das bzw. die wasserlösliche(n) Proteinhydrolysat(e) oder dessen bzw deren wasserlösliche(s) Alkali- und/oder (gegebenenfalls substituierte) Ammonium- und/oder Erdalkalisalz(e) Phenylalanin, vorzugsweise in Mengen von 2 bis 10 g/100 g Protein, besonders bevorzugt von 3 bis 7 g/100 g Protein und insbesondere von 4 bis 6 g/100 g Protein enthalten.

Erfindungsgemäße Verfahren, bei denen das bzw. die wasserlösliche(n) Proteinhydrolysat(e) oder dessen bzw deren wasserlösliche(s) Alkali- und/oder (gegebenenfalls substituierte) Ammonium- und/oder Erdalkalisalz(e) Leucin, vorzugsweise in Mengen von 5 bis 15 g/100 g Protein, besonders bevorzugt von 6,5 bis 12 g/100 g Protein und insbesondere von 7,5 bis 10 g/100 g Protein enthalten, sind ebenfalls bevorzugt.

Erfindungsgemäße Verfahren, bei denen das bzw. die wasserlösliche(n) Proteinhydrolysat(e) oder dessen bzw deren wasserlösliche(s) Alkali- und/oder (gegebenenfalls substituierte) Ammonium- und/oder Erdalkalisalz(e) Isoleucin, vorzugsweise in Mengen von 2 bis 10 g/100 g Protein, besonders bevorzugt von 3 bis 7 g/100 g Protein und insbesondere von 4 bis 6 g/100 g Protein enthalten, sind ebenfalls bevorzugt.

Erfindungsgemäße Verfahren, bei denen das bzw. die wasserlösliche(n) Proteinhydrolysat(e) oder dessen bzw deren wasserlösliche(s) Alkali- und/oder (gegebenenfalls substituierte) Ammonium- und/oder Erdalkalisalz(e) Threonin, vorzugsweise in Mengen von 2 bis 10 g/100 g Protein, besonders bevorzugt von 2,5 bis 7 g/100 g Protein und insbesondere von 3 bis 5,5 g/100 g Protein enthalten, sind ebenfalls bevorzugt.

Erfindungsgemäße Verfahren, bei denen das bzw. die wasserlösliche(n) Proteinhydrolysat(e) oder dessen bzw. deren wasserlösliche(s) Alkali- und/oder (gegebenenfalls substituierte) Ammonium- und/oder Erdalkalisalz(e) Methionin, vorzugsweise in Mengen von 1 bis 10 g/100 g Protein, besonders bevorzugt von 1,5 bis 5 g/100 g Protein und insbesondere von 2 bis 4 g/100 g Protein enthalten, sind ebenfalls bevorzugt.

Erfindungsgemäße Verfahren, bei denen das bzw. die wasserlösliche(n) Proteinhydrolysat(e) oder dessen bzw. deren wasserlösliche(s) Alkali- und/oder (gegebenenfalls substituierte) Ammonium- und/oder Erdalkalisalz(e) Lysin, vorzugsweise in Mengen von 2 bis 12 g/100 g Protein, besonders bevorzugt von 4 bis 9 g/100 g Protein und insbesondere von 6 bis 8 g/100 g Protein enthalten, sind ebenfalls bevorzugt.

Erfindungsgemäße Verfahren, bei denen das bzw. die wasserlösliche(n) Proteinhydrolysat(e) oder dessen bzw. deren wasserlösliche(s) Alkali- und/oder (gegebenenfalls substituierte) Ammonium- und/oder Erdalkalisalz(e) Valin, vorzugsweise in Mengen von 2 bis 10 g/100 g Protein, besonders bevorzugt von 3 bis 7,5 g/100 g Protein und insbesondere von 5 bis 6,5 g/100 g Protein enthalten, sind ebenfalls bevorzugt.

Erfindungsgemäße Verfahren, bei denen das bzw. die wasserlösliche(n) Proteinhydrolysat(e) oder dessen bzw. deren wasserlösliche(s) Alkali- und/oder (gegebenenfalls substituierte) Ammonium- und/oder Erdalkalisalz(e) Histidin, vorzugsweise in Mengen von 1,2 bis 10 g/100 g Protein, besonders bevorzugt von 1,7 bis 5 g/100 g Protein und insbesondere von 2 bis 4 g/100 g Protein enthalten, sind ebenfalls bevorzugt.

Erfindungsgemäße Verfahren, bei denen das bzw. die wasserlösliche(n) Proteinhydrolysat(e) oder dessen bzw. deren wasserlösliche(s) Alkali- und/oder (gegebenenfalls substituierte) Ammonium- und/oder Erdalkalisalz(e) Arginin, vorzugsweise in Mengen von 2 bis 10 g/100 g Protein, besonders bevorzugt von 2,3 bis 7 g/100 g Protein und insbesondere von 3 bis 6 g/100 g Protein enthalten, sind ebenfalls bevorzugt.

Erfindungsgemäße Verfahren, bei denen das bzw. die wasserlösliche(n) Proteinhydrolysat(e) oder dessen bzw. deren wasserlösliche(s) Alkali- und/oder (gegebenenfalls substituierte) Ammonium- und/oder Erdalkalisalz(e) Prolin, vorzugsweise in Mengen von 5 bis 15 g/100 g Protein, besonders bevorzugt von 6 bis 12 g/100 g Protein und insbesondere von 8 bis 10,5 g/100 g Protein enthalten, sind ebenfalls bevorzugt.

Erfindungsgemäße Verfahren, bei denen das bzw. die wasserlösliche(n) Proteinhydrolysat(e) oder dessen bzw deren wasserlösliche(s) Alkali- und/oder (gegebenenfalls substituierte) Ammonium- und/oder Erdalkalisalz(e) Alanin, vorzugsweise in Mengen von 1,5 bis 10 g/100 g Protein, besonders bevorzugt von 2 bis 7 g/100 g Protein und insbesondere von 2,5 bis 4 g/100 g Protein enthalten, sind ebenfalls bevorzugt.

Erfindungsgemäße Verfahren, bei denen das bzw. die wasserlösliche(n) Proteinhydrolysat(e) oder dessen bzw. deren wasserlösliche(s) Alkali- und/oder (gegebenenfalls substituierte) Ammonium- und/oder Erdalkalisalz(e) Alanin, vorzugsweise in Mengen von 1,2 bis 10 g/100 g Protein, besonders bevorzugt von 1,3 bis 7 g/100 g Protein und insbesondere von 2 bis 4 g/100 g Protein enthalten, sind ebenfalls bevorzugt.

Erfindungsgemäße Verfahren, bei denen das bzw. die wasserlösliche(n) Proteinhydrolysat(e) oder dessen bzw. deren wasserlösliche(s) Alkali- und/oder (gegebenenfalls substituierte) Ammonium- und/oder Erdalkalisalz(e) Asparaginsäure, vorzugsweise in Mengen von 2 bis 10 g/100 g Protein, besonders bevorzugt von 3 bis 8 g/100 g Protein und insbesondere von 5 bis 7 g/100 g Protein enthalten, sind ebenfalls bevorzugt.

Erfindungsgemäße Verfahren, bei denen das bzw. die wasserlösliche(n) Proteinhydrolysat(e) oder dessen bzw. deren wasserlösliche(s) Alkali- und/oder (gegebenenfalls substituierte) Ammonium- und/oder Erdalkalisalz(e) Serin, vorzugsweise in Mengen von 2 bis 10 g/100 g Protein, besonders bevorzugt von 3 bis 7 g/100 g Protein und insbesondere von 4 bis 6 g/100 g Protein enthalten, sind ebenfalls bevorzugt.

Erfindungsgemäße Verfahren, bei denen das bzw. die wasserlösliche(n) Proteinhydrolysat(e) oder dessen bzw. deren wasserlösliche(s) Alkali- und/oder (gegebenenfalls substituierte) Ammonium- und/oder Erdalkalisalz(e) Glutaminsäure, vorzugsweise in Mengen von 10 bis 30 g/100 g Protein, besonders bevorzugt von 15 bis 25 g/100 g Protein und insbesondere von 18 bis 22 g/100 g Protein enthalten, sind ebenfalls bevorzugt.

Erfindungsgemäße Verfahren, bei denen das bzw. die wasserlösliche(n) Proteinhydrolysat(e) oder dessen bzw. deren wasserlösliche(s) Alkali- und/oder (gegebenenfalls substituierte) Ammonium- und/oder Erdalkalisalz(e) Glycin, vorzugsweise in Mengen von 1 bis 5 g/100 g Protein, besonders bevorzugt von 1,3 bis 3 g/100 g Protein und insbesondere von 1,5 bis 2,5 g/100 g Protein enthalten, sind ebenfalls bevorzugt.

Erfindungsgemäße Verfahren, bei denen das bzw. die wasserlösliche(n) Proteinhydrolysat(e) oder dessen bzw. deren wasserlösliche(s) Alkali- und/oder (gegebenenfalls substituierte) Ammonium- und/oder Erdalkalisalz(e) Tyrosin, vorzugsweise in Mengen von 2 bis 10 g/100 g Protein, besonders bevorzugt von 3 bis 7 g/100 g Protein und insbesondere von 4 bis 6 g/100 g Protein enthalten, sind ebenfalls bevorzugt.

Zusammenfassend sind erfindungsgemäße Verfahren bevorzugt, bei denen die Proteinhydrolysate mindestens eine der genannten Aminosäuren in den genannten Mengen enthalten. Besonders bevorzugt sind Verfahren, bei denen die Proteinhydrolysate mehrere der genannten Aminosäuren in den genannten Mengen enthalten. Besonders bevorzugte Verfahren sind dadurch gekennzeichnet, daß die Proteinhydrolysate
- Phenylalanin, vorzugsweise in Mengen von 2 bis 10 g/100 g Protein, besonders bevorzugt von 3 bis 7 g/100 g Protein und insbesondere von 4 bis 6 g/100 g Protein und/oder
- Leucin, vorzugsweise in Mengen von 5 bis 15 g/100 g Protein, besonders bevorzugt von 6,5 bis 12 g/100 g Protein und insbesondere von 7,5 bis 10 g/100 g Protein und/oder
- Isoleucin, vorzugsweise in Mengen von 2 bis 10 g/100 g Protein, besonders bevorzugt von 3 bis 7 g/100 g Protein und insbesondere von 4 bis 6 g/100 g Protein und/oder
- Threonin, vorzugsweise in Mengen von 2 bis 10 g/100 g Protein, besonders bevorzugt von 2,5 bis 7 g/100 g Protein und insbesondere von 3 bis 5,5 g/100 g Protein und/oder
- Methionin, vorzugsweise in Mengen von 1 bis 10 g/100 g Protein, besonders bevorzugt von 1,5 bis 5 g/100 g Protein und insbesondere von 2 bis 4 g/100 g Protein und/oder
- Lysin, vorzugsweise in Mengen von 2 bis 12 g/100 g Protein, besonders bevorzugt von 4 bis 9 g/100 g Protein und insbesondere von 6 bis 8 g/100 g Protein und/oder
- Valin, vorzugsweise in Mengen von 2 bis 10 g/100 g Protein, besonders bevorzugt von 3 bis 7,5 g/100 g Protein und insbesondere von 5 bis 6,5 g/100 g Protein und/oder
- Histidin, vorzugsweise in Mengen von 1,2 bis 10 g/100 g Protein, besonders bevorzugt von 1,7 bis 5 g/100 g Protein und insbesondere von 2 bis 4 g/100 g Protein und/oder
- Arginin, vorzugsweise in Mengen von 2 bis 10 g/100 g Protein, besonders bevorzugt von 2,3 bis 7 g/100 g Protein und insbesondere von 3 bis 6 g/100 g Protein und/oder
- Prolin, vorzugsweise in Mengen von 5 bis 15 g/100 g Protein, besonders bevorzugt von 6 bis 12 g/100 g Protein und insbesondere von 8 bis 10,5 g/100 g Protein und/oder
- Alanin, vorzugsweise in Mengen von 1,5 bis 10 g/100 g Protein, besonders bevorzugt von 2 bis 7 g/100 g Protein und insbesondere von 2,5 bis 4 g/100 g Protein und/oder
- Asparaginsäure, vorzugsweise in Mengen von 2 bis 10 g/100 g Protein, besonders bevorzugt von 3 bis 8 g/100 g Protein und insbesondere von 5 bis 7 g/100 g Protein und/oder
- Serin, vorzugsweise in Mengen von 2 bis 10 g/100 g Protein, besonders bevorzugt von 3 bis 7 g/100 g Protein und insbesondere von 4 bis 6 g/100 g Protein und/oder
- Glutaminsäure, vorzugsweise in Mengen von 10 bis 30 g/100 g Protein, besonders bevorzugt von 15 bis 25 g/100 g Protein und insbesondere von 18 bis 22 g/100 g Protein und/oder
- Glycin, vorzugsweise in Mengen von 1 bis 5 g/100 g Protein, besonders bevorzugt von 1,3 bis 3 g/100 g Protein und insbesondere von 1,5 bis 2,5 g/100 g Protein und/oder
- Tyrosin, vorzugsweise in Mengen von 2 bis 10 g/100 g Protein, besonders bevorzugt von 3 bis 7 g/100 g Protein und insbesondere von 4 bis 6 g/100 g Protein
enthalten, wobei insbesondere bevorzugte Verfahren dadurch gekennzeichnet sind, daß die Proteinhydrolysate
- Phenylalanin, vorzugsweise in Mengen von 2 bis 10 g/100 g Protein, besonders bevorzugt von 3 bis 7 g/100 g Protein und insbesondere von 4 bis 6 g/100 g Protein und
- Leucin, vorzugsweise in Mengen von 5 bis 15 g/100 g Protein, besonders bevorzugt von 6,5 bis 12 g/100 g Protein und insbesondere von 7,5 bis 10 g/100 g Protein und
- Isoleucin, vorzugsweise in Mengen von 2 bis 10 g/100 g Protein, besonders bevorzugt von 3 bis 7 g/100 g Protein und insbesondere von 4 bis 6 g/100 g Protein und
- Threonin, vorzugsweise in Mengen von 2 bis 10 g/100 g Protein, besonders bevorzugt von 2,5 bis 7 g/100 g Protein und insbesondere von 3 bis 5,5 g/100 g Protein und
- Methionin, vorzugsweise in Mengen von 1 bis 10 g/100 g Protein, besonders bevorzugt von 1,5 bis 5 g/100 g Protein und insbesondere von 2 bis 4 g/100 g Protein und
- Lysin, vorzugsweise in Mengen von 2 bis 12 g/100 g Protein, besonders bevorzugt von 4 bis 9 g/100 g Protein und insbesondere von 6 bis 8 g/100 g Protein und
- Valin, vorzugsweise in Mengen von 2 bis 10 g/100 g Protein, besonders bevorzugt von 3 bis 7,5 g/100 g Protein und insbesondere von 5 bis 6,5 g/100 g Protein und
- Histidin, vorzugsweise in Mengen von 1,2 bis 10 g/100 g Protein, besonders bevorzugt von 1,7 bis 5 g/100 g Protein und insbesondere von 2 bis 4 g/100 g Protein und
- Arginin, vorzugsweise in Mengen von 2 bis 10 g/100 g Protein, besonders bevorzugt von 2,3 bis 7 g/100 g Protein und insbesondere von 3 bis 6 g/100 g Protein und
- Prolin, vorzugsweise in Mengen von 5 bis 15 g/100 g Protein, besonders bevorzugt von 6 bis 12 g/100 g Protein und insbesondere von 8 bis 10,5 g/100 g Protein und
- Alanin, vorzugsweise in Mengen von 1,5 bis 10 g/100 g Protein, besonders bevorzugt von 2 bis 7 g/100 g Protein und insbesondere von 2,5 bis 4 g/100 g Protein und
- Asparaginsäure, vorzugsweise in Mengen von 2 bis 10 g/100 g Protein, besonders bevorzugt von 3 bis 8 g/100 g Protein und insbesondere von 5 bis 7 g/100 g Protein und
- Serin, vorzugsweise in Mengen von 2 bis 10 g/100 g Protein, besonders bevorzugt von 3 bis 7 g/100 g Protein und insbesondere von 4 bis 6 g/100 g Protein und
- Glutaminsäure, vorzugsweise in Mengen von 10 bis 30 g/100 g Protein, besonders bevorzugt von 15 bis 25 g/100 g Protein und insbesondere von 18 bis 22 g/100 g Protein und
- Glycin, vorzugsweise in Mengen von 1 bis 5 g/100 g Protein, besonders bevorzugt von 1,3 bis 3 g/100 g Protein und insbesondere von 1,5 bis 2,5 g/100 g Protein und
- Tyrosin, vorzugsweise in Mengen von 2 bis 10 g/100 g Protein, besonders bevorzugt von 3 bis 7 g/100 g Protein und insbesondere von 4 bis 6 g/100 g Protein
enthalten.

Wie bereits erwähnt, wird in bevorzugten erfindungsgemäßen Verfahren im Anschluß an das Ausspülen der Anwendungszubereitung mit einer Zubereitung D vor- oder nachgespült. Hier sind erfindungsgemäße Verfahren bevorzugt, bei denen die Zubereitung D bezogen auf ihr Gewicht 2,5 bis 30 Gew.-%, vorzugsweise 5 bis 20 Gew.- % und insbesondere 7,5 bis 15 Gew.-% Calcium- und/oder Aluminiumsalze enthält, wobei bevorzugte Salze ausgewählt sind aus CaCl₂, Al₂(SO₄)₃, NaAl(SO₄)₂, KAl(SO₄)₂, Aluminiumlactat und deren Mischungen.

Neben den genannten Calcium- und/oder Aluminiumsalzen kann die Zubereitung D auch andere Salze von Calcium und/oder Aluminium oder auch Salze von Zink und/oder Seltenerdmetallen enthalten. Bevorzugte Vertreter dieser Gruppe sind beispielsweise Zinksulfat, Zinkacetat sowie die Chloride und Sulfate der Seltenerdmetalle. Zu den Seltenerdmetallen zählen die Elemente Cer, Praseodym, Neodym, Promethium, Samarium, Europium, Gadolinium, Terbium, Dysprosium, Holmium, Erbium, Thulium, Ytterbium und Lutetium.

Weitere bevorzugte Salze stammen aus der Gruppe der Salze von Calcium und/oder Aluminium und/oder Zink und oder Cer und/oder Praseodym und/oder Neodym und/oder Promethium und/oder Samarium und/oder Europium und/oder Gadolinium und/oder Terbium und/oder Dysprosium und/oder Holmium und/oder Erbium und/oder Thulium und/oder Ytterbium und/oder Lutetium mit mindestens einer monomeren und/oder polymeren organischen Säure aus der Gruppe der unverzweigten gesättigten oder ungesättigten Monocarbonsäuren, der verzweigten gesättigten oder ungesättigten Monocarbonsäuren, der gesättigten und ungesättigten Dicarbonsäuren, der unverzweigten oder verzweigten, ungesättigten oder gesättigten, ein- oder mehrfach hydroxylierten Fettsäuren mit mindestens 8 Kohlenstoffatomen, der Harzsäuren, der aromatischen Mono-, Di- und Tricarbonsäuren, der Zuckersäuren, der Hydroxysäuren, der Oxosäuren, der Aminosäuren und/oder der polymeren Carbonsäuren.

Die betreffenden Säuren stammen dabei vorzugsweise. aus der Gruppe der unverzweigten gesättigten oder ungesättigten Monocarbonsäuren, der verzweigten gesättigten oder ungesättigten Monocarbonsäuren, der gesättigten und ungesättigten Dicarbonsäuren, der aromatischen Mono-, Di- und Tricarbonsäuren, der Zuckersäuren, der Hydroxysäuren, der Oxosäuren, der Aminosäuren und/oder der polymeren Carbonsäuren, der unverzweigten oder verzweigten, ungesättigten oder gesättigten, ein- oder mehrfach hydroxylierten Fettsäuren mit mindestens 8 Kohlenstoffatomen und/oder Harzsäuren.

Obwohl erfindungsgemäß alle Salze monomerer und/oder polymerer organischer Säuren in der Zubereitung D enthalten sein können, werden doch, wie vorstehend beschrieben, die Salze monomerer und/oder polymerer organischer Säuren aus den Gruppen der unverzweigten gesättigten oder ungesättigten Monocarbonsäuren, der verzweigten gesättigten oder ungesättigten Monocarbonsäuren, der gesättigten und ungesättigten Dicarbonsäuren, der aromatischen Mono-, Di- und Tricarbonsäuren, der Zuckersäuren, der Hydroxysäuren, der Oxosäuren, der Aminosäuren und/oder der polymeren Carbonsäuren bevorzugt. Innerhalb dieser Gruppen werden im Rahmen der vorliegenden Erfindung wiederum die in der Folge genannten Säuren bevorzugt:

Aus der Gruppe der unverzweigten gesättigten oder ungesättigten Monocarbonsäuren: Methansäure (Ameisensäure), Ethansäure (Essigsäure), Propansäure (Propionsäure), Pentansäure (Valeriansäure), Hexansäure (Capronsäure), Heptansäure (Önanthsäure), Octansäure (Caprylsäure), Nonansäure (Pelargonsäure), Decansäure (Caprinsäure), Undecansäure, Dodecansäure (Laurinsäure), Tridecansäure, Tetradecansäure (Myristinsäure), Pentadecansäure, Hexadecansäure (Palmitinsäure), Heptadecansäure (Margarinsäure), Octadecansäure (Stearinsäure), Eicosansäure (Arachinsäure), Docosansäure (Behensäure), Tetracosansäure (Lignocerinsäure), Hexacosansäure (Cerotinsäure), Triacotansäure (Melissinsäure), 9c-Hexadecensäure (Palmitoleinsäure), 6c-Octadecensäure (Petroselinsäure), 6t-Octadecensäure (Petroselaidinsäure), 9c-Octadecensäure (Ölsäure), 9t-Octadecensäure (Elaidinsäure), 9c,12c-Octadecadiensäure (Linolsäure), 9t,12t-Octadecadiensäure (Linolaidinsäure) und 9c,12c,15c-Octadecatreinsäure (Linolensäure).

Aus der Gruppe der verzweigten gesättigten oder ungesättigten Monocarbonsäuren: 2-Methylpentansäure, 2-Ethylhexansäure, 2-Propylheptansäure, 2-Butyloctansäure, 2-Pentylnonansäure, 2-Hexyldecansäure, 2-Heptylundecansäure, 2-Octyldodecansäure, 2-Nonyltridecansäure, 2-Decyltetradecansäure, 2- Undecylpentadecansäure, 2-Dodecylhexadecansäure, 2-Tridecylheptadecansäure, 2-Tetradecyloctadecansäure, 2-Pentadecylnonadecansäure, 2-Hexadecyleicosansäure, 2-Heptadecylheneicosansäure.

Aus der Gruppe der unverzweigten gesättigten oder ungesättigten Di- oder Tricarbonsäuren: Propandisäure (Malonsäure), Butandisäure (Bernsteinsäure), Pentandisäure (Glutarsäure), Hexandisäure (Adipinsäure), Heptandisäure (Pimelinsäure), Octandisäure (Korksäure), Nonandisäure (Azelainsäure), Decandisäure (Sebacinsäure), 2c-Butendisäure (Maleinsäure), 2t-Butendisäure (Fumarsäure), 2-Butindicarbonsäure (Acetylendicarbonsäure).

Aus der Gruppe der aromatischen Mono-, Di- und Tricarbonsäuren: Benzoesäure, 2-Carboxybenzoesäure (Phthalsäure), 3-Carboxybenzoesäure (Isophthalsäure), 4-Carboxybenzoesäure (Terephthalsäure), 3,4-Dicarboxybenzoesäure (Trimellithsäure), 3,5-Dicarboxybenzoesäure (Trimesionsäure).

Aus der Gruppe der Zuckersäuren: Galactonsäure, Mannonsäure, Fructonsäure, Arabinonsäure, Xylonsäure, Ribonsäure, 2-Desoxy-ribonsäure, Alginsäure.

Aus der Gruppe der Hydroxysäuren: Hydroxyphenylessigsäure (Mandelsäure), 2-Hydroxypropionsäure (Milchsäure), Hydroxybernsteinsäure (Äpfelsäure), 2,3-Dihydorxybutandisäure (Weinsäure), 2-Hydroxy-1,2,3-propantricarbonsäure (Citronensäure), Ascorbinsäure, 2-Hydroxybenzoesäure (Salicylsäure), 3,4,5-Trihydroxybenzoesäure (Gallussäure).

Aus der Gruppe der Oxosäuren: 2-Oxopropionsäure (Brenztraubensäure), 4-Oxopentansäure (Lävulinsäure).

Aus der Gruppe der Aminosäuren: Alanin, Valin, Leucin, Isoleucin, Prolin, Tryptophan, Phenylalanin, Methionin, Glycin, Serin, Tyrosin, Threonin, Cystein, Asparagin, Glutamin, Asparaginsäure, Glutaminsäure, Lysin, Arginin, Histidin.

Aus der Gruppe der polymeren Carbonsäuren: Polyacrylsäure, Polymethacrylsäure, Alkylacrylamid/Acrylsäure-Copolymere, Alkylacrylamid/Methacryisäure-Copolymere, Alkylacrylamid/Methylmethacrylsäure-Copolymere, Copolymere aus ungesättigten Carbonsäuren, Vinylacetat/Crotonsäure-Copolymere, VinylpyrrolidonNinylacrylat-Copolymere.

Bevorzugt sind beispielsweise das Zinkformiat, das Zinkacetat, das Zinklactat, das Zinktosylat (p-Toluolsulfonsäure-Zn-Salz) und das Zinkgluconat sowie die entsprechenden Calcium- und/oder Aluminium- und/oder Selenerdmetallsalze.

### Weitere Bestandteile

Die im erfindungsgemäßen Verfahren eingesetzten Zusammensetzungen können in einer bevorzugten Ausführungsform mindestens ein Alkalisierungsmittel enthalten. Erfindungsgemäß können die dem Fachmann für Bleich- bzw. Aufhellmittel bekannten, üblichen Alkalisierungsmittel wie Ammonium-, Alkalimetall- und Erdalkalimetallhydroxide, -carbonate, -hydrogencarbonate, -hydroxycarbonate und -carbamide, sowie Alkaliphosphate und Alkalimetasilikate sowie Ammoniak und Alkalihydroxide verwendet werden. Einsetzbar sind erfindungsgemäß aber auch die organischen Amine, wie beispielsweise Monoethanolamin, Arginin, Lysin, Monoethanolamin, Monoisopropanolamin, 2-Amino-2-methylpropanol, 2-Amino-2-methyl-1,3-propandiol, 2-Amino-2-ethyl-1,3-propandiol und 2-Amino-2-methylbutanol.

Die im erfindungsgemäßen Verfahren eingesetzten Zusammensetzungen enthalten die Alkalisierungsmittel bevorzugt in Mengen von 0.2 bis 25 Gew.-%, insbesondere 0.5 bis 10 Gew.-%.

Weiterhin kann es erfindungsgemäß bevorzugt sein, wenn die im erfindungsgemäßen Verfahren eingesetzten Zusammensetzungen mindestens ein Tensid enthalten, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in im erfindungsgemäßen Verfahren eingesetzten Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- anionische Alkyl- und/oder Alkenyl-Oligoglykosidcarboxylate, -sulfate, -phosphate und/oder -isethionate, die sich von Alkyl- und/oder Alkenyloligoglykosiden der allgemeinen Formel (IV) ableiten,

   R-O-(G)ₚ (IV)

   mit der Bedeutung
   R C₆₋₂₂-Alkyl oder C₆₋₂₂-Alkenyl,
   G Glykosideinheit, die sich von einem Zucker mit 5 oder 6 Kohlenstoffatomen ableitet,
   p Zahl von 1 bis 10.

Bevorzugt sind Carboxylate, wie sie beispielsweise unter dem Handelsnamen Plantapon® LGC (Alkylpolyglucosid-Carboxylat Natriumsalz; 30 % Aktivsubstanz; Fa. Cognis) vermarktet werden
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate, anionische Alkyl- und/oder Alkenyl-Oligoglykosidcarboxylate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungsprodukte von Ethylenoxid an Sorbitanfettsäureester
- Anlagerungsprodukte von Ethylenoxid an Fettsäurealkanolamide.

Beispiele für die in den im erfindungsgemäßen Verfahren eingesetzten Aufhellmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid^{®}S 18 erhältliche Stearylamidopropyldimethylamin, zeichnen sich neben einer guten konditionierenden Wirkung speziell durch ihre gute biologische Abbaubarkeit aus. Ebenfalls sehr gut biologisch abbaubar sind quaternäre Esterverbindungen, sogenannte "Esterquats", wie die unter dem Warenzeichen Stepantex^{®} vertriebenen Methylhydroxyalkyldialkoyloxyalkyl-ammoniummethosulfate sowie die unter dem Warenzeichen Dehyquart^{®} vertriebenen Produkte wie Dehyquart^{®} AU-46.

Ein Beispiel für ein als kationisches Tensid einsetzbares quaternäres Zuckerderivat stellt das Handelsprodukt Glucquat^{®}100 dar, gemäß INCI-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

In einer weiteren Ausführungsform enthalten die im erfindungsgemäßen Verfahren eingesetzten Aufhellmittel mindestens einen strukturverbessernden Wirkstoff.

Solche die Haarstruktur verbessernden Wirkstoffe stellen Vitamine und deren Derivate beziehungsweise Vorstufen dar. Erfindungsgemäß besonders bevorzugt sind Panthenol und seine physiologisch verträglichen Derivate. Solche Derivate sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 A1 offenbarten kationischen Panthenolderivate. Ein erfindungsgemäß bevorzugtes Panthenolderivat ist ferner dessen Vorstufe Pantolacton. Panthenol ist innerhalb dieser Gruppe bevorzugt. Ein weiteres Beispiel für ein strukturverbesserndes Vitamin ist Pyridoxin (Vitamin B6).

Weiterhin ist auch Polyvinylpyrrolidon (PVP) für seine faserstrukturverbessernden Eigenschaften bekannt und erfindungsgemäß bevorzugt.

Weitere, erfindungsgemäß besonders wirksame, strukturverbessernde Verbindungen stellen die Aldehyde dar. Besonders bevorzugte Beispiel sind Formaldehyd und Formaldehyd-abspaltende Verbindungen, wie beispielsweise Methoxymethylester, Dimethylol (thio) harnstoffderivate, Oxazolidinderivate, N-Hydroxymethylmaleinimid, Hexamethylentetramin und seine Derivate, Hydantoinderivate, Pyridinium-substituierte Dimethylether, Imidazolidinylharnstoff-Derivate, Isothiazolinone, 2-Brom-2-nitropropandiol und 5-Brom-5-nitro-1,3-dioxan. Weitere besonders bevorzugte Aldehyde sind Acetaldehyd, Glyoxal, Glycerinaldehyd und Glutardialdehyd.

Eine weitere geeignete Gruppe von strukturverbessernden Wirkstoffen sind Pflanzenextrakte.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, grünem Tee, Ginseng und Ingwerwurzel bevorzugt.

Besonders bevorzugt sind die Extrakte aus Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, grünem Tee, Ginseng und Ingwerwurzel.

Ganz besonders für die im erfindungsgemäßen Verfahren eingesetzten Aufhellmittel geeignet sind die Extrakte aus Mandel, Aloe Vera, Kokosnuß, Mango, Aprikose, Limone, Weizen, Kiwi, Melone und grünem Tee.

Wenn die Pflanzenextrakte in die wasserfreie Zusammensetzung des ersten Erfindungsgegenstandes eingearbeitet werden, ist es sinnvoll, die wässrigen Lösemittel zu entfernen.

Weitere strukturverbessernde Wirkstoffe sind quaternisierte Proteinhydrolysate. Besonders bevorzugt sind stark abgebaute Keratinhydrolysate mit Molmassen im Bereich von 400 bis 800. Ferner sind quaternierte Proteinhydrolysate, wie sie beispielsweise unter den Handelsbezeichnungen Gluadin^{®} WQ (INCI-Bezeichnung: Laurdimonium Hydroxypropyl Hydrolyzed Wheat Protein) und Crotein^{®} Q (INCI-Bezeichnung: Hydroxypropyltrimonium Hydrolyzed Collagen) vertrieben werden, erfindungsgemäß besonders bevorzugt. Wenn die Proteinhydrolysate in die wasserfreie Zusammensetzung des ersten Erfindungsgegenstandes eingearbeitet werden, ist es sinnvoll, die wässrigen Lösemittel zu entfernen.

Neben den quaternierten Proteinhydrolysaten stellen auch quaternäre Polymere erfindungsgemäß bevorzugte strukturverbessernde Verbindungen dar. Besonders bevorzugt sind die Polymere, die unter den Handelsbezeichnungen Mirapol^{®} A15 (INCI-Bezeichnung: Polyquaternium-2), Onamer^{®} M (INCI-Bezeichnung: Polyquaternium-1) und Merquat^{®} 100 (INCI-Bezeichnung: Polyquaternium-6), Polymer JR 400 (INCI-Bezeichnung: Polyquaternium-10) vertrieben werden.

Ebenfalls faserstrukturverbessernde Wirkstoffe sind Mono-, Di- und Oligosaccharide wie beispielsweise Glucose, Galactose, Fructose, Fruchtzucker, Saccharose und Lactose. Weiterhin können auch Derivate dieser Pentosen, Hexosen und Heptosen, wie die entsprechenden On- und Uronsäuren (Zuckersäuren), Zuckeralkohole, Zuckeramine, wie beispielsweise N-Glucosamin, und Glykoside, sowie mit C₄-C₃₀-Fettalkoholen veretherte Pentosen, Hexosen und Heptosen, erfindungsgemäß eingesetzt werden. Die Zuckersäuren können erfindungsgemäß in freier Form, in Form ihrer Salze, bevorzugt sind Calcium-, Magnesium- und Zink-Salze, und in Form ihrer Ester oder Lactone eingesetzt werden. Bevorzugte Zuckersäuren sind die Gluconsäure, Gluconsäure-γ-lacton, Lactobionsäure, die Glucuronsäure und ihre Mono- beziehungsweise Dilactone, die Pangaminsäure, die Zuckersäure, die Mannozuckersäure und ihre Monobeziehungsweise Dilactone sowie die Schleimsäure und ihre Mono- beziehungsweise Dilactone. Bevorzugte Zuckeralkohole sind Sorbit, Mannit und Dulcit. Bevorzugte Glykoside sind die Methylglucoside. Glucose, N-Glucosamin und Gluconsäure sind aus dieser Gruppe besonders bevorzugt.

Auch gewisse Aminosäuren sind in Rahmen der vorliegenden Erfindung als haarstrukturverbessernde Wirkstoffe einsetzbar. Beispiele sind die in der DE-195 22 569, auf die hier ausdrücklich Bezug genommen wird, beschriebenen Aminosäuren Serin, Threonin und Tyrosin. Ferner sind auch Derivate des Serins, wie beispielsweise das Serinphosphat, erfindungsgemäß bevorzugt. Eine weitere strukturverbessernde Aminosäure stellt Lysin dar. Serin ist ein besonders bevorzugter faserstrukturverbessernder Wirkstoff.

Ebenfalls zur Strukturverbesserung können bestimmte Säuren, insbesondere von Komponente (A2) verschiedene α-Hydroxycarbonsäuren, und ihre Salze eingesetzt werden. Erfindungsgemäß bevorzugte strukturverbessernde Säuren sind Milchsäure, Äpfelsäure, Weinsäure, Glycerinsäure und Maleinsäure. Milchsäure und Glycerinsäure sind besonders bevorzugt. Weiterhin verbessern spezielle Phosphonsäuren und ihre Salze die Struktur keratinhaltiger Fasern. Erfindungsgemäß bevorzugte Phosphonsäuren sind die n-Octylphosphonsäure und die n-Decylphosphonsäure.

Weiterhin kann als strukturverbessernder Wirkstoff Vitamin B₃ eingesetzt werden. Unter dieser Bezeichnung werden häufig die Verbindungen Nicotinsäure und Nicotinsäureamid (Niacinamid) geführt. Erfindungsgemäß bevorzugt ist das Nicotinsäureamid.

Auch Vitamin H ist als strukturverbessernder Wirkstoff im Sinne der vorliegenden Erfindung einsetzbar. Als Vitamin H wird die Verbindung (3aS,4S, 6aR)-2-Oxohexahydrothienol[3,4-*d*]-imidazol-4-valeriansäure bezeichnet, für die sich aber zwischenzeitlich der Trivialname Biotin durchgesetzt hat.

Erfindungsgemäß besonders bevorzugte strukturverbessernde Wirkstoffe sind ausgewählt aus Panthenol, physiologisch verträglichen Panthenol-Derivaten, Mono-, Di- und Oligosacchariden, Serin, Milchsäure, Glycerinsäure, Niacinamid, Vitamin B6, Polyvinylpyrrolidon, Glucose, Gluconsäure und Biotin.

Die im erfindungsgemäßen Verfahren eingesetzten Mittel enthalten die strukturverbessernden Wirkstoffe bevorzugt in Mengen von 0,1 bis 5 Gew.-%, besonders bevorzugt in Mengen von 0,2 bis 2 Gew.-%.

Die im erfindungsgemäßen Verfahren eingesetzten Mittel können weiterhin alle für solche Zubereitungen bekannten Wirk-, Zusatz- und Hilfsstoffe enthalten.

Weiterhin können die im erfindungsgemäßen Verfahren eingesetzten Aufhellmittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten.
Bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate.
- polymere Dimethyldiallylammoniumsalze und deren Copolymere mit Acrylsäure sowie Estern und Amiden von Acrylsäure und Methacrylsäure. Die unter den Bezeichnungen Merquat^{®}100 (Poly(dimethyldiallylammoniumchlorid)), Merquat^{®}550 (Dimethyldiallylammoniumchlorid-Acrylamid-Copolymer) und Merquat^{®} 280 (Dimethyldiallylammoniumchlorid-Acrylsäure-Copolymer im Handel erhältlichen Produkte sind Beispiele für solche kationischen Polymere.
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat^{®}734 und Gafquat^{®}755 im Handel erhältlich.
- Vinylpyrrolidon-Methoimidazoliniumchlorid-Copolymere, wie sie unter der Bezeichnung Luviquat^{®} angeboten werden.
- quaternierter Polyvinylalkohol
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen, ganz besonders bevorzugt sind Polyquaternium-2, Polyquaternium-10 und Polyquaternium-22.

Als konditionierende Wirkstoffe weiterhin geeignet sind Silikonöle, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl, sowie Tocopherolacetat.

Gleichfalls geeignete haarkonditionierende Verbindungen sind Phospholipide, beispielsweise Sojalecithin, Ei-Lecithin und Kephaline.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyltrimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle,
- Cyclodextrine,
- Lösungsmittel und -vermittler wie Ethanol, Isopropanol, Ethylenglykol, Propylenglykol, Glycerin, Dimethylisosorbid und Diethylenglykol,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Das erfindungsgemäße Verfahren zur Blondierung (Aufhellung) keratinischer Fasern kann selbstverständlich mit einer Färbung der Fasern verbunden werden, indem Farbstoffe und/oder deren Vorprodukte in die Anwendungsmischung inkorporiert werden. In dieser Anwendungsform handelt es sich bei dem erfindungsgemäßen Blondierverfahren um ein Verfahren zum Färben und gleichzeitigen Aufhellen keratinischer Fasern.

In einem solchen erfindungsgemäßen Verfahren eingesetzte Anwendungsmischungen enthalten vorzugsweise mindestens eine Kuppler- und mindestens eine Entwicklerkomponente. Kuppler- und Entwicklerkomponenten werden auch als Oxidationsfarbstoffvorprodukte bezeichnet. Zudem können die im erfindungsgemäßen Verfahren eingesetzten Anwendungsmischungen auch noch direktziehende Farbstoffe als Nuanceure enthalten.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.
Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 2-Amino-4-(2-hydroxyethylamino)-anisol (Lehmanns Blau), 1-Phenyl-3-methyl-pyrazol-5-on, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Hinsichtlich in den im erfindungsgemäßen Verfahren eingesetzten Anwendungsmischungen einsetzbaren weiteren Farbstoffvorprodukte unterliegt die vorliegende Erfindung keinerlei Einschränkungen. Die erfindungsgemäßen Färbemittel können als weitere Farbstoffvorprodukte
- Oxidationsfarbstoffvorprodukte vom Entwickler- und/oder Kuppler-Typ, und
- Vorstufen naturanaloger Farbstoffe, wie Indol- und Indolin-Derivate,
sowie Mischungen von Vertretern dieser Gruppen enthalten.

In einer ersten bevorzugten Ausführungsform enthalten die im erfindungsgemäßen Verfahren eingesetzten Anwendungsmischungen weiterhin mindestens eine Entwicklerkomponente. Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterozyklische Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 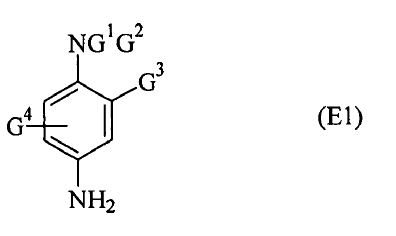 wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, . der mit einer stickstoffhaltigen Gruppe substituiert ist;
- G³ steht für ein Wasserstoffatom, ein Halogenatom, wie ein Chlor-, Brom-, lod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄- Mesylaminoalkoxyrest oder einen C₁- bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für die als Substituenten genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)amino-2-methylanilin, 4-N,N-Bis-(β-hydroxyethyl)amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den im erfindungsgemäßen Verfahren eingesetzten Anwendungsmischungen verwendet werden können, kann man insbesondere die Verbindungen nennen, die der folgenden Formel (E2) entsprechen, sowie ihre physiologisch verträglichen Salze: 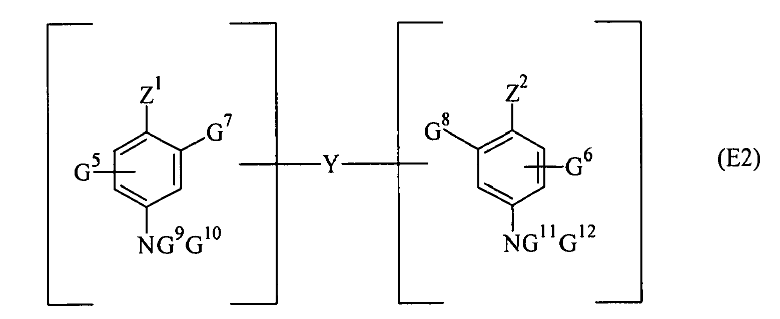 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest,
mit den Maßgaben, dass
- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4'-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 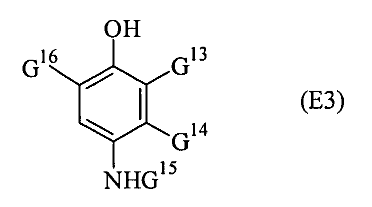 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁- bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁ bis C₄)-alkylrest, einen C₁- bis C₄-Aminoalkylrest oder einen C₁- bis C₄-Cyanoalkylrest,
- G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(□-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethylaminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterozyklischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen 2,5-Diamino-pyridin, 2-(4'-Methoxyphenyl)amino-3-amino-pyridin, 2,3-Diamino-6-methoxy-pyridin, 2-(β-Methoxyethyl)amino-3-amino-6-methoxy-pyridin und 3,4-Diamino-pyridin.

Bevorzugte Pyrimidin-Derivate sind insbesondere 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)amino-1-methylpyrazol.

Bevorzugte Pyrazol-Pyrimidin-Derivate sind insbesondere die Derivate des Pyrazol-[1,5-a]-pyrimidin der folgenden Formel (E4) und dessen tautomeren Formen, sofern ein tautomeres Gleichgewicht besteht: 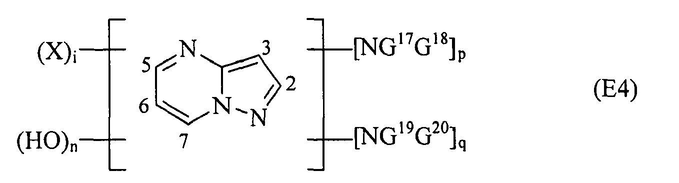 wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁- bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄-hydroxyalkyl)aminoalkylrest, einen Aminorest, einen C₁- bis C₄-Alkyl- oder Di-(C₁- bis C₄-hydroxyalkyl)aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, dass
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazol-[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

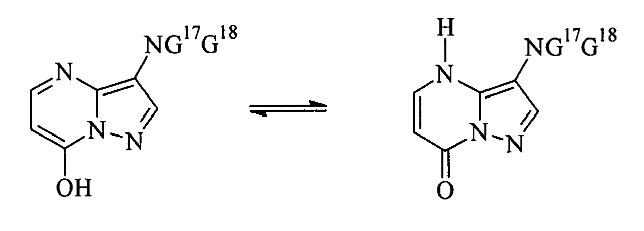

Unter den Pyrazol-[1,5-a]-pyrimidinen der obenstehenden Formel (E4) kann man insbesondere nennen:
- Pyrazol-[1,5-a]-pyrimidin-3,7-diamin;
- 2,5-Dimethyl-pyrazol-[1,5-a]-pyrimidin-3,7-diamin;
- Pyrazol-[1,5-a]-pyrimidin-3,5-diamin;
- 2,7-Dimethyl-pyrazol-[1,5-a]-pyrimidin-3,5-diamin;
- 3-Aminopyrazol-[1,5-a]-pyrimidin-7-ol;
- 3-Aminopyrazol-[1,5-a]-pyrimidin-5-ol;
- 2-(3-Aminopyrazol-[1,5-a]-pyrimidin-7-ylamino)-ethanol;
- 2-(7-Aminopyrazol-[1,5-a]-pyrimidin-3-ylamino)-ethanol;
- 2-[(3-Aminopyrazol-[1,5-a]-pyrimidin-7-yl)-(2-hydroxy-ethyl)amino]-ethanol;
- 2-[(7-Aminopyrazol-[1,5-a]-pyrimidin-3-yl)-(2-hydroxy-ethyl)amino]-ethanol;
- 5,6-Dimethylpyrazol-[1,5-a]-pyrimidin-3,7-diamin;
- 2,6-Dimethylpyrazol-[1,5-a]-pyrimidin-3,7-diamin;
- 3-Amino-7-dimethylamino-2,5-dimethylpyrazol-[1,5-a]-pyrimidin;
sowie ihre physiologisch verträglichen Salze und ihre tautomeren Formen, wenn ein tautomeres Gleichgewicht vorhanden ist.

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

Erfindungsgemäß besonders bevorzugte Verfahren sind dadurch gekennzeichnet, daß die Entwicklerkomponente ausgewählt ist aus 3-Methyl-1,4-diaminobenzol, 1-(2'-Hydroxyethyl)-2,5-diaminobenzol, 2-(2,5-Diaminophenoxy)-ethanol, N,N-Bis(2'-Hydroxyethyl)-1,4-diaminobenzol, 3-Methyl-4-aminophenol und 2-Methylamino-4-aminophenol, p-Phenylendiamin, 2-(β-Hydroxyethyl)-p- phenylendiamin, N,N-Bis-(β-hydroxyethyl)-p-phenylendiamin, N,N'-bis-(β- Hydroxyethyl)-N,N'-bis- (4'-aminophenyl)-1,3-diamino-propan-2-ol, Bis-(2-hydroxy-5- aminophenyl)- methan, N,N'-Bis-(4'-aminophenyl)-1,4-diazacycloheptan, 1,10-Bis-(2',5'- diaminophenyl)-1,4,7,10-tetraoxadecan, p-Aminophenol, 4- Amino-3-fluorphenol, 4-Amino-2-aminomethylphenol, 4- Amino-2- ((diethylamino)methyl)phenol, o-Aminophenol, 2-Amino-4- methylphenol, 2-Amino-5- methylphenol, 2-Amino-4-chlorphenol, 2,4,5,6- Tetraaminopyrimidin, 4-Hydroxy-2,5,6- triaminopyrimidin, 2-Hydroxy-4,5,6- triaminopyrimidin, 2-Dimethylamino-4,5,6- triaminopyrimidin, 2,4- Dihydroxy-5,6-diaminopyrimidin, 2,5,6-Triaminopyrimidin, 1-(2'- Hydroxy- 5'-aminobenzyl)-imidazolidin-2-on, 4,5-Diamino-1-(2'-hydroxyethyl) pyrazol und N-(4-Amino-3-methylphenyl)-N-[3-(1 H-imidazol-1-yl)propyl]amin trihydrochlorid.

Die in den im erfindungsgemäßen Verfahren eingesetzten Anwendungsmischungen enthalten vorzugsweise mindestens eine Kupplerkomponente.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol und 1-Amino-3-bis-(2'-hydroxyethyl)aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)amino-3,4-methylendioxybenzol.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders gut als Vorstufen naturanaloger Haarfarbstoffe geeignet sind Derivate des 5,6-Dihydroxyindolins der Formel (VIIIa), 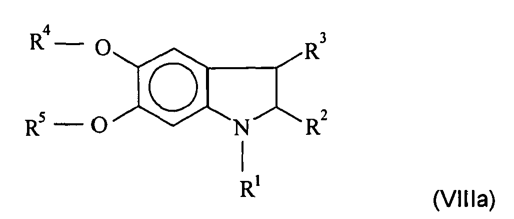 in der unabhängig voneinander
- R¹ steht für Wasserstoff, eine C₁-C₄-Alkylgruppe oder eine C₁-C₄-Hydroxy-alkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (Vlllb), 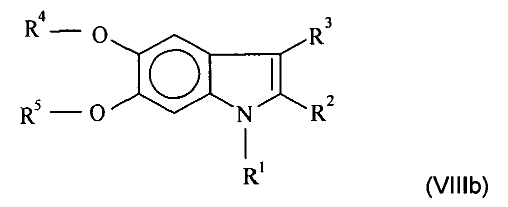 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den im Rahmen des erfindungsgemäßen Verfahrens eingesetzten Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

In einer weiteren Ausführungsform kann es erfindungsgemäß bevorzugt sein, das Indolin- oder Indolderivat in Haarfärbemitteln in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin und Histidin, insbesondere Arginin.

Erfindungsgemäß besonders bevorzugte Verfahren sind dadurch gekennzeichnet, daß die Kupplerkomponente ausgewählt ist aus m-Phenylendiaminderivaten, Naphtholen, Resorcin und Resorcinderivaten, Pyrazolonen, m-Aminophenolen und substituierten Pyridinderivaten, wobei bevorzugte Mittel Resorcin, 3-Amino-2-methylamino-6-methoxypyridin, 3-Amino-6-methylphenol, 3-Amino-2-hydroxypyridin, 1,3-Bis-(2,4-diaminophenoxy)propan, 2,7-Dihydroxynaphthalin, 2-Methylresorcin, 5-Methyl-resorcin, 2,5-Dimethylresorcin und 4-Chlorresorcin 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin, 2,6-Dihydroxy-3,4-dimethylpyridin, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 3-Amino-4-(2-methoxyethoxy)-5-methylphenylamin und/oder 2-[(3-Morpholin-4-ylphenyl)amino]ethanol dihydrochlorid enthalten.

Vorzugsweise werden Kuppler- und Entwicklerkomponenten in einem bestimmten Verhältnis zueinander eingesetzt. Hier sind erfindungsgemäße im erfindungsgemäßen Verfahren eingesetzte Anwendungsmischungen bevorzugt, die die Kupplerkomponente(n) in einer Menge von 0,01 bis 20 Gew.-%, vorzugsweise von 0,5 bis 5 Gew.-%, und die Entwicklerkomponente(n) in einer Menge von 0,01 bis 20 Gew.-%, vorzugsweise von 0,5 bis 5 Gew.-%, jeweils bezogen auf die gesamte Anwendungsmischung, enthalten.

Zusätzlich können die im erfindungsgemäßen Verfahren eingesetzten Anwendungsmischungen zur Nuancierung einen oder mehrere direktziehende Farbstoffe enthalten. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)amino-2-nitrobenzol, 3-Nitro-4-(β-hydroxyethyl)aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Erfindungsgemäß bevorzugte Verfahren sind dadurch gekennzeichnet, daß die Anwendungsmischungen einen direktziehenden Farbstoff enthalten, der ausgewählt ist aus Nitrophenylendiaminen, Nitroaminophenolen, Azofarbstoffen, Anthrachinonen oder Indophenolen, vorzugsweise aus der Gruppe der unter den internationalen Bezeichnungen bzw. Handelsnamen bekannten Farbstoffe HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2-Hydroxyethyl)amino-4,6-dinitrophenol, 1-(2'-Hydroxyethyl)amino-4-methyl-2-nitrobenzol, 1-Amino-4-(2-hydroxyethyl)-amino-5-chlor-2-nitrobenzol, 4-Amino-3-nitrophenol, 1-(2-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die im erfindungsgemäßen Verfahren eingesetzten Anwendungsmischungen einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterozyklus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

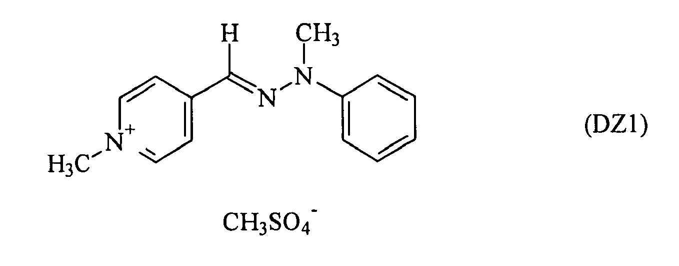

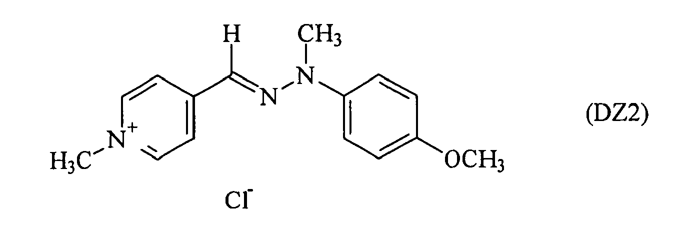

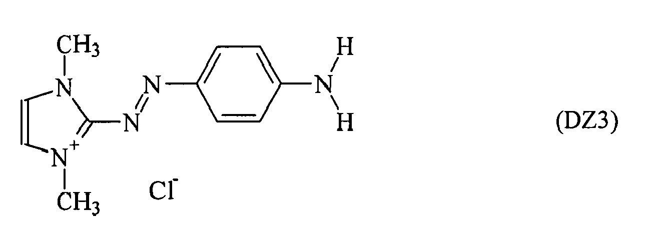

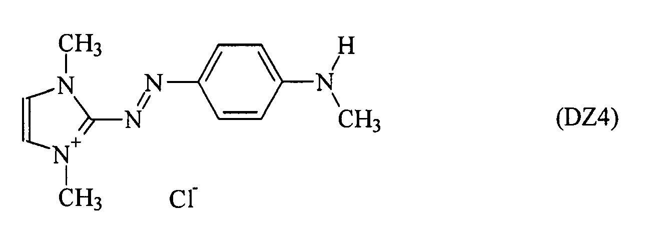

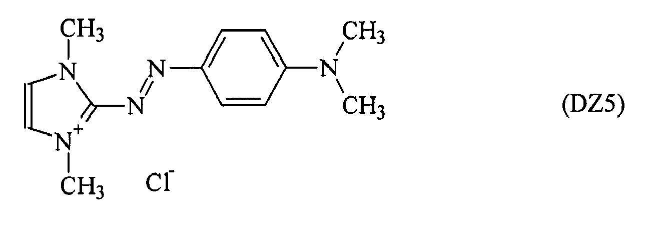

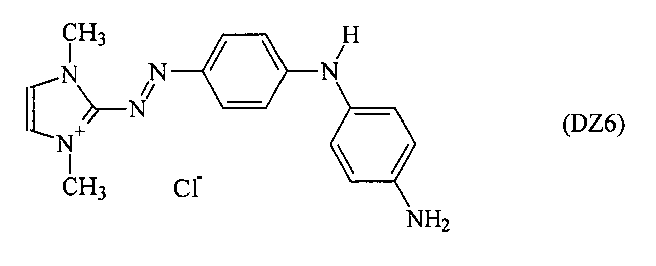

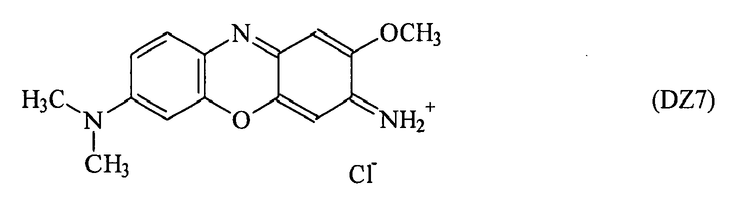

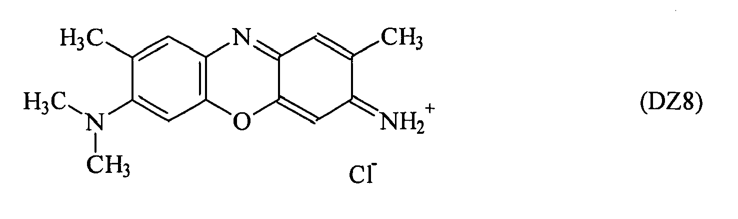

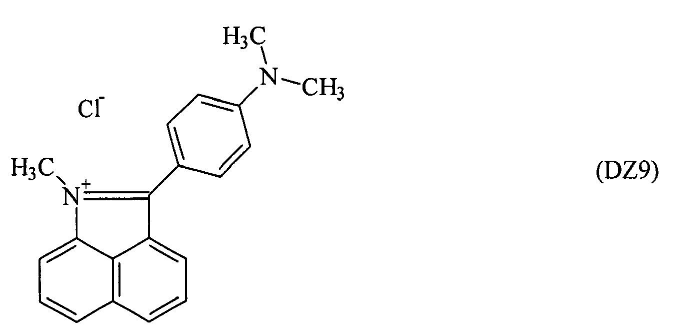

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Die im erfindungsgemäßen Verfahren eingesetzten Anwendungsmischungen gemäß dieser Ausführungsform enthalten die direktziehenden Farbstoffe bevorzugt in einer Menge von 0,01 bis 20 Gew.-%, bezogen auf das gesamte Färbemittel.

Weiterhin können die im erfindungsgemäßen Verfahren eingesetzten Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den im erfindungsgemäßen Verfahren eingesetzten Anwendungsmischungen einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

In einer weiteren Ausführungsform enthalten die im erfindungsgemäßen Verfahren eingesetzten Anwendungsmischungen eine Kombination aus Komponente
A Verbindungen, die eine reaktive Carbonylgruppe enthalten mit Komponente
B Verbindungen, ausgewählt aus (a) CH-aciden Verbindungen, (b) Verbindungen mit primärer oder sekundärer Aminogruppe oder Hydroxygruppe, ausgewählt aus primären oder sekundären aromatischen Aminen, stickstoffhaltigen heterozyklischen Verbindungen und aromatischen Hydroxyverbindungen, (c) Aminosäuren, (d) aus 2 bis 9 Aminosäuren aufgebauten Oligopeptiden.

Erfindungsgemäße Verbindungen mit einer reaktiven Carbonylgruppe (im Folgenden auch reaktive Carbonylverbindungen oder Komponente A genannt) besitzen mindestens eine Carbonylgruppe als reaktive Gruppe, welche mit den Verbindungen der Komponente B unter Ausbildung einer beide Komponenten verknüpfenden chemischen Bindung reagiert. Ferner sind erfindungsgemäß auch solche Verbindungen als Komponente A umfaßt, in denen die reaktive Carbonylgruppe derart derivatisiert bzw. maskiert ist, daß die Reaktivität des Kohlenstoffatoms der derivatisierten bzw. maskierten Carbonylgruppe gegenüber der Komponente B stets vorhanden ist. Diese Derivate sind bevorzugt Kondensationsverbindungen von reaktiven Carbonylverbindungen mit
a) Aminen und deren Derivate unter Bildung von Iminen oder Oximen als Kondensationsverbindung
b) von Alkoholen unter Bildung von Acetalen oder Ketalen als Kondensationsverbindung
c) von Wasser unter Bildung von Hydraten als Additionsverbindung.

Die Komponente A wird bevorzugt ausgewählt aus der Gruppe, die gebildet wird aus Acetophenon, Propiophenon, 2-Hydroxyacetophenon, 3-Hydroxyacetophenon, 4-Hydroxyacetophenon, 2-Hydroxypropiophenon, 3-Hydroxypropiophenon, 4-Hydroxypropiophenon, 2-Hydroxybutyrophenon, 3-Hydroxybutyrophenon, 4-Hydroxybutyrophenon, 2,4-Dihydroxyacetophenon, 2,5-Dihydroxyacetophenon, 2,6-Dihydroxyacetophenon, 2,3,4-Trihydroxyacetophenon, 3,4,5-Trihydroxyacetophenon, 2,4,6-Trihydroxyacetophenon, 2,4,6-Trimethoxyacetophenon, 3,4,5-Trimethoxyacetophenon, 3,4,5-Trimethoxyacetophenon-diethylketal, 4-Hydroxy-3-methoxy-acetophenon, 3,5-Dimethoxy-4-hydroxyacetophenon, 4-Aminoacetophenon, 4-Dimethylaminoacetophenon, 4-Morpholinoacetophenon, 4-Piperidinoacetophenon, 4-Imidazolinoacetophenon, 2-Hydroxy-5-brom-acetophenon, 4-Hydroxy-3-nitroacetophenon, Acetophenon-2-carbonsäure, Acetophenon-4-carbonsäure, Benzophenon, 4-Hydroxybenzophenon, 2-Aminobenzophenon, 4,4'-Dihydroxybenzophenon, 2,4-Dihydroxy-benzophenon, 2,4,4'-Trihydroxybenzophenon, 2,3,4-Trihydroxybenzophenon, 2-Hydroxy-1-acetonaphthon, 1-Hydroxy-2-acetonaphthon, Chromon, Chromon-2-carbonsäure, Flavon, 3-Hydroxyflavon, 3,5,7-Trihydroxyflavon, 4',5,7-Trihydroxyflavon, 5,6,7-Trihydroxyflavon, Quercetin, 1-Indanon, 9-Fluorenon, 3-Hydroxyfluorenon, Anthron, 1,8-Dihydroxyanthron, Vanillin, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxybenzaldehyd, 4-Hydroxy-2,6-dimethoxy-benzaldehyd, 4-Hydroxy-2-methyl-benzaldehyd, 4-Hydroxy-3-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 4-Hydroxy-3,5-dimethoxybenzaldehyd, 4-Hydroxy-3,5-dimethyl-benzaldehyd, 3,5-Diethoxy-4-hydroxybenzaldehyd, 2,6-Diethoxy-4-hydroxy-benzaldehyd, 3-Hydroxy-4-methoxy-benzaldehyd, 2-Hydroxy-4-methoxy-benzaldehyd, 2-Ethoxy-4-hydroxy-benzaldehyd, 3-Ethoxy-4-hydroxy-benzaldehyd, 4-Ethoxy-2-hydroxy-benzaldehyd, 4-Ethoxy-3-hydroxybenzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,5-Dihydroxybenzaldehyd, 2,6-Dihydroxybenzaldehyd, 3,4-Dihydroxybenzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Hydroxy-2-methoxybenzaldehyd, 4-Dimethylaminobenzaldehyd, 4-Diethylaminobenzaldehyd, 4-Dimethylamino-2-hydroxybenzaldehyd, 4-Diethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Hydroxy-1-naphthaldehyd, 4-Dimethylamino-1-naphthaldehyd, 2-Methoxy-zimtaldehyd, 4-Methoxy-zimtaldehyd, 4-Hydroxy-3-methoxy-zimtaldehyd, 3,5-Dimethoxy-4-hydroxyzimtaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylamino-benzaldehyd, 4-Diphenylamino-benzaldehyd, 4-Dimethylamino-2-methoxybenzaldehyd, 4-(1-Imidazolyl)-benzaldehyd, Piperonal, 2,3,6,7-Tetrahydro-1 H,5H-benzo[ij]chinolizin-9-carboxaldehyd, 2,3,6,7-Tetrahydro-8-hydroxy-1 H,5H-benzo[ij]chinolizin-9-carboxaldehyd, N-Ethylcarbazol-3-aldehyd, 2-Formylmethylen-1,3,3-trimethylindolin (Fischers Aldehyd oder Tribasen Aldehyd), 2-Indolaldehyd, 3-Indolaldehyd, 1-Methylindol-3-aldehyd, 2-Methylindol-3-aldehyd, 1-Acetylindol-3-aldehyd, 3-Acetylindol, 1-Methyl-3-acetylindol, 2-(1',3',3'-Trimethyl-2-indolinyliden)-acetaldehyd, 1-Methylpyrrol-2-aldehyd, 1-Methyl-2-acetylpyrrol, 4-Pyridinaldehyd, 2-Pyridinaldehyd, 3-Pyridinaldehyd, 4-Acetylpyridin, 2-Acetylpyridin, 3-Acetylpyridin, Pyridoxal, Chinolin-3-aldehyd, Chinolin-4-aldehyd, Antipyrin-4-aldehyd, Furfural, 5-Nitrofurfural, 2-Thenoyl-trifluor-aceton, Chromon-3-aldehyd, 3-(5'-Nitro-2'-furyl)-acrolein, 3-(2'-Furyl)-acrolein und Imidazol-2-aldehyd, 1,3-Diacetylbenzol, 1,4-Diacetylbenzol, 1,3,5-Triacetylbenzol, 2-Benzoyl-acetophenon, 2-(4'-Methoxybenzoyl)-acetophenon, 2-(2'-Furoyl)-acetophenon, 2-(2'-Pyridoyl)-acetophenon und 2-(3'-Pyridoyl)-acetophenon, Benzylidenaceton, 4-Hydroxybenzylidenaceton, 2-Hydroxybenzylidenaceton, 4-Methoxybenzylidenaceton, 4-Hydroxy-3-methoxybenzylidenaceton, 4-Dimethylaminobenzylidenaceton, 3,4-Methylendioxybenzylidenaceton, 4-Pyrrolidinobenzylidenaceton, 4-Piperidinobenzylidenaceton, 4-Morpholinobenzylidenaceton, 4-Diethylaminobenzylidenaceton, 3-Benzyliden-2,4-pentandion, 3-(4'-Hydroxybenzyliden)-2,4-pentandion, 3-(4'-Dimethylaminobenzyliden)-2,4-pentandion, 2-Benzylidencyclohexanon, 2-(4'-Hydroxybenzyliden)-cyclohexanon, 2-(4'-Dimethylaminobenzyliden)-cyclohexanon, 2-Benzyliden-1,3-cyclohexandion, 2-(4'-Hydroxybenzyliden)-1,3-cyclohexandion, 3-(4'-Dimethylaminobenzyliden)-1,3-cyclohexandion, 2-Benzyliden-5,5-dimethyl-1,3-cyclohexandion, 2-(4'-Hydroxybenzyliden)-5,5-dimethyl-1,3-cyclohexandion, 2-(4'-Hydroxy-3-methoxybenzyliden)-5,5-dimethyl-1,3-cyclohexandion, 2-(4'-Dimethylaminobenzyliden)-5,5-dimethyl-1,3-cyclohexandion, 2-Benzylidencyclopentanon, 2'-(4-Hydroxybenzyliden)-cyclopentanon, 2-(4'-Dimethylaminobenzyliden)-cyclopentanon, 5-(4-Dimethylaminophenyl)penta-2,4-dienal, 5-(4-Diethylaminophenyl)penta-2,4-dienal, 5-(4-Methoxyphenyl)penta-2,4-dienal, 5-(3,4-Dimethoxyphenyl)penta-2,4-dienal, 5-(2,4-Dimethoxyphenyl)penta-2,4-dienal, 5-(4-Piperidinophenyl)penta-2,4-dienal, 5-(4-Morpholinophenyl)penta-2,4-dienal, 5-(4-Pyrrolidinophenyl)penta-2,4-dienal, 6-(4-Dimethylaminophenyl)hexa-3,5-dien-2-on, 6-(4-Diethylaminophenyl)hexa-3,5-dien-2-on, 6-(4-Methoxyphenyl)hexa-3,5-dien-2-on, 6-(3,4-Dimethoxyphenyl)hexa-3,5-dien-2-on, 6-(2,4-Dimethoxyphenyl)hexa-3,5-dien-2-on, 6-(4-Piperidinophenyl)hexa-3,5-dien-2-on, 6-(4-Morpholinophenyl)hexa-3,5-dien-2-on, 6-(4-Pyrrolidinophenyl)hexa-3,5-dien-2-on, 5-(4-Dimethylamino-1-naphthyl)penta-3,5-dienal, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 4-Hydroxy-3-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 6-Nitropiperonal, 2-Nitropiperonal, 5-Nitrovanillin, 2,5-Dinitrosalicylaldehyd, 5-Brom-3-nitrosalicylaldehyd, 3-Nitro-4-formylbenzolsulfonsäure, 4-Nitro-1-naphthaldehyd, 2-Nitrozimtaldehyd, 3-Nitrozimtaldehyd, 4-Nitrozimtaldehyd, 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd, 4-Formyl-1-methylpyridinium-, 2-Formyl-1-methylpyridinium-, 4-Formyl-1-ethylpyridinium- , 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 4-Acetyl-1-methylpyridinium-, 2-Acetyl-1-methylpyridinium-, 4-Acetyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzylchinolinium-, 6-Formyl-1-benzylchinolinium-, 7-Formyl-1-benzylchinolinium-, 8-Formyl-1-benzylchinolinium, 5-Formyl-1-allylchinolinium-, 6-Formyl-1-allylchinolinium-, 7-Formyl-1-allylchinolinium- und 8-Formyl-1-allylchinolinium-, 5-Acetyl-1-methylchinolinium-, 6-Acetyl-1-methylchinolinium-, 7-Acetyl-1-methylchinolinium-, 8-Acetyl-1-methylchinolinium, 5-Acetyl-1-ethylchinolinium-, 6-Acetyl-1-ethylchinolinium-, 7-Acetyl-1-ethylchinolinium-, 8-Acetyl-1-ethylchinolinium, 5-Acetyl-1-benzylchinolinium-, 6-Acetyl-1-benzylchinolinium-, 7-Acetyl-1-benzylchinolinium-, 8-Acetyl-1-benzylchinolinium, 5-Acetyl-1-allylchinolinium-, 6-Acetyl-1-allylchinolinium-, 7-Acetyl-1-allylchinolinium- und 8-Acetyl-1-allylchinolinium, 9-Formyl-10-methylacridinium-, 4-(2'-Formylvinyl)-1-methylpyridinium-, 1,3-Dimethyl-2-(4'-formylphenyl)-benzimidazolium-, 1,3-Dimethyl-2-(4'-formylphenyl)-imidazolium-, 2-(4'-Formylphenyl)-3-methylbenzothiazolium-, 2-(4'-Acetylphenyl)-3-methylbenzothiazolium-, 2-(4'-Formylphenyl)-3-methylbenzoxazolium-, 2-(5'-Formyl-2'-furyl)-3-methylbenzothiazolium-, 2-(5'-Formyl-2'-furyl)-3-methylbenzothiazolium-, 2-(5'-Formyl-2'-thienyl)-3-methylbenzothiazolium-, 2-(3'-Formylphenyl)-3-methylbenzothiazolium-, 2-(4'-Formyl-1-naphthyl)-3-methylbenzothiazolium-, 5-Chlor-2-(4'-formylphenyl)-3-methylbenzothiazolium-, 2-(4'-Formylphenyl)-3,5-dimethylbenzothiazolium-benzolsulfonat, -p-toluolsulfonat, -methansulfonat, -perchlorat, -sulfat, -chlorid, -bromid, -iodid, -tetrachlorozinkat, -methylsulfat-, trifluormethansulfonat, -tetrafluoroborat, Isatin, 1-Methyl-isatin, 1-Allyl-isatin, 1-Hydroxymethyl-isatin, 5-Chlor-isatin, 5-Methoxyisatin, 5-Nitroisatin, 6-Nitro-isatin, 5-Sulfo-isatin, 5-Carboxy-isatin, Chinisatin, 1-Methylchinisatin, sowie beliebigen Gemischen der voranstehenden Verbindungen.

Ganz besonders bevorzugt werden in den im erfindungsgemäßen Verfahren eingesetzten Anwendungsmischungen Benzaldehyd, Zimtaldehyd und Naphthaldehyd sowie deren Derivate, insbesondere mit einem oder mehreren Hydroxy-, Alkoxy- oder Aminosubstituenten, als reaktive Carbonylverbindung verwendet. Dabei werden wiederum die Verbindungen gemäß Formel (Ca-1) bevorzugt, 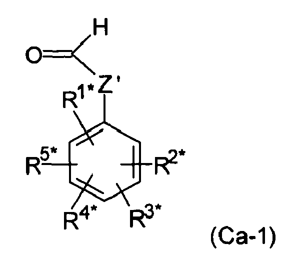 worin
- R^{1*}, R^{2*} und R^{3*} stehen unabhängig voneinander für ein Wasserstoffatom, ein Halogenatom, eine C₁-C₆-Alkylgruppe, eine Hydroxygruppe, eine C₁-C₆-Alkoxygruppe, eine C₁-C₆-Dialkylaminogruppe, eine Di(C₂-C₆-hydroxyalkyl)aminogruppe, eine Di(C₁-C₆-alkoxy-C₁-C₆-alkyl)aminoguppe, eine C₁-C₆-Hydroxyalkyloxygruppe, eine Sulfonylgruppe, eine Carboxygruppe, eine Sulfonsäuregruppe, eine Sulfonamidogruppe, eine Sulfonamidgruppe, eine Carbamoylgruppe, eine C₂-C₆-Acylgruppe oder eine Nitrogruppe,
- Z' steht für eine direkte Bindung oder eine Vinylengruppe,
- R^{4*} und R^{5*} stehen für ein Wasserstoffatom oder bilden gemeinsam, zusammen mit dem Restmolekül einen 5- oder 6-gliederigen aromatischen oder aliphatischen Ring.

Die Derivate der Benzaldehyde, Naphthaldehyde bzw. Zimtaldehyde der reaktiven Carbonylverbindung gemäß Komponente C werden besonders bevorzugt ausgewählt aus bestimmten Aldehyden. Hier sind im erfindungsgemäßen Verfahren eingesetzte Anwendungsmischungen bevorzugt, die zusätzlich mindestens eine reaktive Carbonylverbindung enthalten, die ausgewählt wird, aus der Gruppe, bestehend aus 4-Hydroxy-3-methoxybenzaldehyd, 3,5-Dimethoxy-4-hydroxybenzaldehyd, 4-Hydroxy-1-naphthaldehyd, 4-Hydroxy-2-methoxybenzaldehyd, 3,4-Dihydroxy-5-methoxybenzaldehyd, 3,4,5-Trihydroxybenzaldehyd, 3,5-Dibrom-4-hydroxybenzaldehyd, 4-Hydroxy-3-nitrobenzaldehyd, 3-Brom-4-hydroxybenzaldehyd, 4-Hydroxy-3-methylbenzaldehyd, 3,5-Dimethyl-4-hydroxy-benzaldehyd, 5-Brom-4-hydroxy-3-methoxybenzaldehyd, 4-Diethylamino-2-hydroxybenzaldehyd, 4-Dimethylamino-2-methoxybenzaldehyd, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxy-benzaldehyd, 4-Hydroxy-2,6-dimethoxy-benzaldehyd, 4-Hydroxy-2-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 3,5-Diethoxy-4-hydroxybenzaldehyd, 2,6-Diethoxy-4-hydroxy-benzaldehyd, 3-Hydroxy-4-methoxy-benzaldehyd, 2-Hydroxy-4-methoxy-benzaldehyd, 2-Ethoxy-4-hydroxy-benzaldehyd, 3-Ethoxy-4-hydroxy-benzaldehyd, 4-Ethoxy-2-hydroxy-benzaldehyd, 4-Ethoxy-3-hydroxybenzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,4-Dihydroxy-3-methyl-benzaldehyd, 2,4-Dihydroxy-5-methylbenzaldehyd, 2,4-Dihydroxy-6-methyl-benzaldehyd, 2,4-Dihydroxy-3-methoxybenzaldehyd, 2,4-Dihydroxy-5-methoxy-benzaldehyd, 2,4-Dihydroxy-6-methoxybenzaldehyd, 2,5-Dihydroxybenzaldehyd, 2,6-Dihydroxybenzaldehyd, 3,4-Dihydroxybenzaldehyd, 3,4-Dihydroxy-2-methyl-benzaldehyd, 3,4-Dihydroxy-5-methylbenzaldehyd, 3,4-Dihydroxy-6-methyl-benzaldehyd, 3,4-Dihydroxy-2-methoxybenzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Dimethylaminobenzaldehyd, 4-Diethylaminobenzaldehyd, 4-Dimethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 3,5-Dichlor-4-hydroxybenzaldehyd, 4-Hydroxy-3,5-düod-benzaldehyd, 3-Chlor-4-hydroxybenzaldehyd, 5-Chlor-3,4-dihydroxybenzaldehyd, 5-Brom-3,4-dihydroxybenzaldehyd, 3-Chlor-4-hydroxy-5-methoxybenzaldehyd, 4-Hydroxy-3-iod-5-methoxybenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Dimethylamino-1-naphthaldehyd, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 6-Nitropiperonal, 2-Nitropiperonal, 5-Nitrovanillin, 2,5-Dinitrosalicylaldehyd, 5-Brom-3-nitrosalicylaldehyd, 4-Nitro-1-naphthaldehyd, 2-Nitrozimtaldehyd, 3-Nitrozimtaldehyd, 4-Nitrozimtaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylamino-benzaldehyd, 4-Diphenylamino-benzaldehyd, 4-(1-Imidazolyl)-benzaldehyd und Piperonal.

Erfindungsgemäß bevorzugt im erfindungsgemäßen Verfahren eingesetzte Anwendungsmischungen sind dadurch gekennzeichnet, daß sie als Haarfärbemittel formuliert sind, die bezogen auf ihr Gewicht 0,01 bis 20 Gew.-%, vorzugsweise 0,05 bis 15 Gew.-%, besonders bevorzugt 0,1 bis 12,5 Gew.-% und insbesondere 0,2 bis 10 Gew.-% einer oder mehrerer reaktive Carbonylverbindung(en) enthalten.

Als CH-acide werden im allgemeinen solche Verbindungen angesehen, die ein an ein aliphatisches Kohlenstoffatom gebundenes Wasserstoffatom tragen, wobei aufgrund von Elektronen-ziehenden Substituenten eine Aktivierung der entsprechenden Kohlenstoff-Wasserstoff-Bindung bewirkt wird. Unter CH-acide Verbindungen fallen erfindungsgemäß auch Enamine, die durch alkalische Behandlung von quaternierten N-Heterozyklen mit einer in Konjugation zum quartären Stickstoff stehenden CH-aciden Alkylgruppe entstehen.

Die CH-aciden Verbindungen der Komponente B sind bevorzugt ausgewählt aus der Gruppe bestehend aus 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 2,3-Dimethyl-naphtho[1,2-d]thiazolium-p-toluolsulfonat, 3-Ethyl-2-methyl-naphtho[1,2-d]thiazolium-p-toluolsulfonat, Rhodanin, Rhodanin-3-essigsäure, 1,4-Dimethylchinolinium-iodid, 1,2-Dimethylchinolinium-iodid, Barbitursäure, Thiobarbitursäure, 1,3-Dimethylthiobarbitursäure, 1,3-Diethylthiobarbitursäure, 1,3-Diethylbarbitursäure, Oxindol, 3-Indoxylacetat, 2-Cumaranon, 5-Hydroxy-2-cumaranon, 6-Hydroxy-2-cumaranon, 3-Methyl-1-phenyl-pyrazolin-5-on, Indan-1,2-dion, Indan-1,3-dion, Indan-1-on, Benzoylacetonitril, 3-Dicyanmethylenindan-1-on, 2-Amino-4-imino-1,3-thiazolin-hydrochlorid, 5,5-Dimethylcyclohexan-1,3-dion, 2H-1,4-Benzoxazin-4H-3-on, 3-Ethyl-2-methyl-benzoxazoliumiodid, 3-Ethyl-2-methyl-benzothiazoliumiodid, 1-Ethyl-4-methyl-chinoliniumiodid, 1-Ethyl-2-methylchinoliniumiodid, 1,2,3-Trimethylchinoxaliniumiodid, 3-Ethyl-2-methyl-benzoxazolium-p-toluolsulfonat, 3-Ethyl-2-methyl-benzothiazolium-p-toluolsulfonat, 1-Ethyl-4-methyl-chinolinium-p-toluolsulfonat, 1-Ethyl-2-methylchinolinium-p-toluolsulfonat, und 1,2,3-Trimethylchinoxalinium-p-toluolsulfonat.

Geeignete Verbindungen mit primärer oder sekundärerAminogruppe als Komponente B sind z.B. primäre aromatische Amine wie N,N-Dimethyl-, N,N-Diethyl-, N- (2-Hydroxyethyl)-N-ethyl-, N,N-Bis-(2-hydroxyethyl)-, N-(2-Methoxyethyl-), 2,3-, 2,4-, 2,5-Dichlor-p-phenylendiamin, 2-Chlor-p-phenylendiamin, 2,5-Dihydroxy-4- morpholinoanilindihydrobromid, 2-, 3-, 4-Aminophenol, 2-Aminomethyl-4-aminophenol, 2- Hydroxymethyl-4-aminophenol, o-, p-Phenylendiamin, o-Toluyiendiamin, 2,5-Diaminotoluol, -phenot, - phenethol, 4-Amino-3-methylphenol, 2-(2,5-Diaminophenyl)-ethanoi, 2,4- Diaminophenoxyethanoi, 2-(2,5-Diaminophenoxy)-ethanol, 4-Methylamino-, 3-Amino-4- (2'-hydroxyethyloxy)-, 3,4-Methylendiamino-, 3,4-Methylendioxyanilin, 3- Amino-2,4-dichlor-, 4-Methylamino-, 2-Methyl-5-amino-, 3-Methyl-4-amino-, 2-Methyl-5-(2- hydroxyethylamino)-, 6-Methyl-3-amino-2-chlor-, 2-Methyl-5-amino-4-chlor-, 3, 4-Methylendioxy-, 5-(2-Hydroxyethylamino)-4-methoxy-2-methyl-, 4-Amino-2- hydroxymethylphenol, 1,3-Diamino-2,4-dimethoxybenzol, 2-, 3-, 4-Aminobenzoesäure, - phenylessigsäure, 2,3-, 2,4-, 2,5-, 3,4-, 3,5-Diaminobenzoesäure, 4-, 5-Aminosalicyisäure, 3- Amino-4-hydroxy-, 4-Amino-3-hydroxy-benzoesäure, 2-, 3-, 4-Aminobenzolsulfonsäure, 3-Amino- 4-hydroxybenzolsulfonsäure, 4-Amino-3-hydroxynaphthalin-1 -sulfonsäure, 6- Amino-7-hydroxynaphthalin-2-sulfonsäure, 7-Amino-4-hydroxynaphthalin-2- sulfonsäure, 4-Amino-5-hydroxynaphthalin-2,7-disulfonsäure, 3-Amino-2-naphthoesäure, 3- Aminophthalsäure, 5-Aminoisophthalsäure, 1,3,5-, 1,2,4-Triaminobenzol, 1,2,4,5- Tetraaminobenzol, 2,4,5-Triaminophenol, Pentaaminobenzol, Hexaaminobenzol, 2,4,6- Triaminoresorcin, 4,5-Diaminobrenzcatechin, 4,6-Diaminopyrogaliol, 3,5-Diamino-4- hydroxybrenzcatechin, aromatische Aniline bzw. Phenole mit einem weiteren aromatischen Rest, wie sie in der nachstehenden Formel dargestellt sind 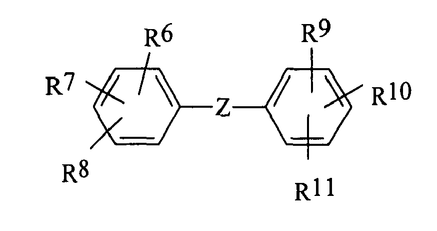 in der R6 für eine Hydroxy- oder eine Aminogruppe, die durch C1-4-Alkyl, Cl.4-Hydroxyalkyl- oderC1-4-Alkoxy-C1-4-alkyl substituiert sein kann, steht, R 7 , R", R9, R'O und RI' für Wasserstoff, eine Hydroxy- oder eine Aminogruppe, die durch eine Cl-4-Alkyl- , Cl-4-Hydroxyalkyl, CI-4-Aminoalkyl- oder CI-4-Alkoxy- C1-4-alkylgruppe substituiert sein kann, für eine Carbon- doer Sulfonsäuregruppe stehen, und Z für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel 111 Q-(CH2-p-CH2-Q')1 > (111) in der P eine direkte Bindung, eine CH2- oder CHOH-Gruppe bedeutet, Q und Q' unabhängig voneinander für ein Sauerstoffatom, eine NR 12 - Gruppe, worin R 12 Wasserstoff, eine Cl-,-Alkyl-, oder Hydroxy-C 1-4-alkylgruppe bedeutet, die Gruppe 0-(CH2)p-NH oder NH-(CH2)p,-0, worin p und p' 2 oder 3 sind, stehen und o eine Zahl von 1 bis 4 bedeutet, wie beispielsweise 4,4'-Diaminostilben, 4,4'-Diaminostilben-2,2'- disulfonsäure-mono- oder -di-Na-Salz, 4-Amino-4'-dimethylaminostilben, 4,4'- Diaminodiphenylmethan, -sulfid, -sulfoxid, -amin, 4,4'-Diaminodiphenylamin-2-sulfonsäure, 4,4'- Diaminobenzophenon, - diphenylether, 3,3',4,4'-Tetraaminodiphenyl, 3,3',4,4'-Tetraamino- benzophenon, 1,3-Bis- (2,4-diaminophenoxy)-propan, 1,8-Bis-(2,5-diaminophenoxy)-3,6-dioxaoctan, 1,3-Bis-(4-aminophenylamino)-propan, -2-propanoi, 1,3-Bis-[N-(4-aminophenyl)-2- hydroxyethylamino]-2-propanol, N,N-Bis-[2-(4-aminophenoxy)-ethyl]-methylamin, N- Phenyl-1,4-phenylendiamin.

Die vorgenannten Verbindungen können sowohl in freier Form als auch in Form ihrer physiologisch verträglichen Salze, insbesondere als Salze anorganischer Säuren, wie Salz- oder Schwefelsäure, eingesetzt werden.

Geeignete stickstoffhaltige heterocyclische Verbindungen sind z.B. 2-, 3-, 4-Amino-, 2-Amino-3-hydroxy-, 2,6-Diamino-, 2,5-Diamino-, 2,3-Diamino-, 2- Dimethylamino-5-amino- , 2-Methylamino-3-amino-6-methoxy-, 2,3-Diamino-6-methoxy-, 2,6-Dimethoxy- 3,5-diamino-, 2,4,5-Triamino-, 2,6-Dihydroxy-3,4-dimethylpyridin, 2,4-Dihydroxy- 5,6-diamino-, 4,5,6-Triamino-, 4-Hydroxy-2,5,6-triamino-, 2-Hydroxy-4,5,6-triamino-, 2, 4,5,6-Tetraamino-, 2-Methylamino-4,5,6-triamino-, 2,4-, 4,5-Diamino-, 2-Amino-4- methoxy-6-methyl- pyrimidin, 3,5-Diaminopyrazol, -1,2,4-triazol, 3-Amino-, 3-Amino-5-hydroxypyrazol, 2-,3-, 8-Aminochinolin, 4-Amino-chinaidin, 2-, 6-Aminonicotinsäure, 5-Aminoisochinolin, 5-, 6- Aminoindazol, 5-, 7-Amino-benzimidazol, -benzothiazol, 2,5-Dihydroxy-4- morpholinoani- lin sowie Indole und Indoline, sowie deren physiologisch verträgliche Salze, sein. Bevorzugte Beispiele für Indol- bzw. Indolinderivate 5,6-Dihydroxyindol, N- Methyl-5,6-dihy- droxyindol, N-Ethyl-5,6-dihydroxyindol, N-Propyl-5,6-dihydroxyindol, N- Butyl-5,6-dihy- droxyindol, 5,6-Dihydroxyindol-2-carbonsäure, 6-Hydroxyindol, 6- Aminoindol und 4-Aminoindol. Weiterhin bevorzugt sind 5,6-Dihydroxyindolin, N-Methyl-5,6- dihydroxyindolin, N-Ethyl-5,6-dihydroxyindolin, N-Propyl-5,6-dihydroxyindolin, N-Butyl-5,6- dihydroxyindolin, 5,6-Dihydroxyindolin-2-carbonsäure. 6-Hydroxyindolin, 6-Aminoindolin und 4-Aminoindolin. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Als Aminosäuren kommen bevorzugt alle natürlich vorkommenden und synthetischen (alpha-Aminosäuren in Frage, z.B. die durch Hydrolyse aus pflanzlichen oder tierischen Proteinen, z.B. Kollagen, Keratin, Casein, Elastin, Sojaprotein, Weizengluten oder Mandelprotein, zugänglichen Aminosäuren. Dabei können sowohl sauer als auch alkalisch reagierende Aminosäuren eingesetzt werden. Bevorzugte Aminosäuren sind Arginin, Histidin, Tyrosin, Phenylalanin, DOPA (Dihydroxyphenylalanin), Ornithin, Lysin und Tryptophan. Es können aber auch andere Aminosäuren verwendet werden, wie z. B. 6- Aminocapronsäure.

Die Oligopeptide können dabei natürlich vorkommende oder synthetische Oligopeptide, aber auch die in Polypeptid- oder Proteinhydrolysaten enthaltenen Oligopeptide sein, sofern sie über eine für die Anwendung in den erfindungsgemäßen Färbemitteln ausreichende Wasserlöslichkeit verfügen. Als Beispiele sind z.B. Glutathion oder die in den Hydrolysaten von Kollagen, Keratin, Casein, Elastin, Sojaprotein, Weizengluten oder Mandelprotein enthaltenen Oligopeptide zu nennen. Bevorzugt ist dabei die Verwendung gemeinsam mit Verbindungen mit primärer oder sekundärer Aminogruppe oder mit aromatischen Hydroxyverbindungen.

Geeignete aromatische Hydroxyverbindungen sind z.B. 2-, 4-, 5- Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2-Hydroxyethyl)-, 3,4-Methylendioxyphenol, 2,4-, 3,4-Dihydroxybenzoesäure, - phenylessigsäure, Gallussäure, 2,4,6-Trihydroxybenzoesäure, -acetophenon, 2-, 4-Chlorresorcin, 1-Naphthol, 1,5-, 2,3-, 2,7-Dihydroxynaphthalin, 6- Dimethylamino-4-hydroxy-2-naphthalinsulfonsäure, 3,6-Dihydroxy-2,7-naphthalinsulfonsäure.

Als CH-aktive Verbindungen können beispielhaft genannt werden 1,2,3,3- Tetramethyl-3H-indoliumiodid, 1,2,3,3-Tetramethyl-3H-indolium-p-toluoisulfonat, 1,2, 3,3-Tetramethyl-3H-indolium-methansulfonat, Fischersche Base (1,3,3-Trimethyl-2- methylenindolin), 2,3-Dimethyl-benzothiazoliumiodid, 2,3-Dimethyl-benzothiazolium-p- toluolsulfonat, Rhodanin, Rhodanin-3-essigsäure, 1 -Ethyl-2-chinaldiniumiodid, 1 -Methyl-2-chinaldiniumiodid Barbitursäure, Thiobarbitursäure, 1,3-Dimethylthiobarbitursäure, Diethylthiobarbitursäure, Oxindol, 3-Indoxylacetat, Cumaranon und 1-Methyl-3-phenyl-2-pyrazolinon.

Bevorzugt werden die CH-aciden Verbindungen aus den Formeln (IX) und/oder (X) und/oder (XI) ausgewählt 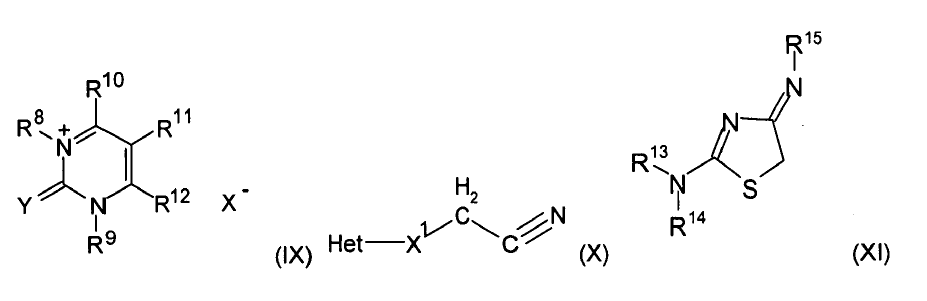 worin
- R⁸ und R⁹ stehen unabhängig voneinander für eine lineare oder cyclische C₁-C₆-Alkylgruppe, eine C₂-C₆-Alkenylgruppe, eine gegebenenfalls substituierte Arylgruppe, eine gegebenenfalls substituierte Heteroarylgruppe, eine Aryl-C₁-C₆-alkylgruppe, eine C₁-C₆-Hydroxyalkylgruppe, eine C₂-C₆-Polyhydroxyalkylgruppe, eine C₁-C₆-Alkoxy-C₁-C₆-alkylgruppe, eine Gruppe R^{I}R^{II}N-(CH₂)ₘ-, worin R^{I} und R^{II} stehen unabhängig voneinander für ein Wasserstoffatom, eine C₁-C₄-Alkylgruppe, eine C₁-C₄-Hydroxyalkylgruppe oder eine Aryl-C₁-C₆-alkylgruppe, wobei R^{I} und R^{II} gemeinsam mit dem Stickstoffatom einen 5-, 6- oder 7-gliedrigen Ring bilden können und m steht für eine Zahl 2, 3, 4, 5 oder 6,
- R¹⁰ und R¹² stehen unabhängig voneinander für ein Wasserstoffatom oder eine C₁-C₆-Alkylgruppe, wobei mindestens einer der Reste R¹⁰ und R¹² eine C₁-C₆-Alkylgruppe bedeutet,
- R¹¹ steht für ein Wasserstoffatom, eine C₁-C₆-Alkylgruppe, eine C₁-C₆-Hydroxyalkylgruppe, eine C₂-C₆-Polyhydroxyalkylgruppe, eine C₁-C₆-Alkoxygruppe, eine C₁-C₆-Hydroxyalkoxygruppe, eine Gruppe R^{III}R^{IV}N-(CH₂)_{q}-, worin R^{III} und R^{IV} stehen unabhängig voneinander für ein Wasserstoffatom, eine C₁-C₆-Alkylgruppe, eine C₁-C₆-Hydroxyalkylgruppe oder eine Aryl-C₁-C₆-alkylgruppe und q steht für eine Zahl 1, 2, 3, 4, 5 oder 6, wobei der Rest R¹¹ zusammen mit einem der Reste R¹⁰ oder R¹² einen 5- oder 6-gliedrigen aromatischen Ring bilden kann, der gegebenenfalls mit einem Halogenatom, einer C₁-C₆-Alkylgruppe, einer C₁-C₆-Hydroxyalkylgruppe, einer C₂-C₆-Polyhydroxyalkylgruppe, einer C₁-C₆-Alkoxygruppe, einer C₁-C₆-Hydroxyalkoxygruppe, einer Nitrogruppe, einer Hydroxygruppe, einer Gruppe R^{V}R^{VI}N-(CH₂)ₛ-, worin R^{V} und R^{VI} stehen unabhängig voneinander für ein Wasserstoffatom, eine C₁-C₆-Alkylgruppe, eine C₁-C₆-Hydroxyalkylgruppe oder eine Aryl-C₁-C₆-alkylgruppe und s steht für eine Zahl 0, 1, 2, 3, 4, 5 oder 6 substituiert sein kann,
- R¹³ und R¹⁴ bilden entweder zusammen mit dem Stickstoffatom einen gesättigten oder ungesättigten 5- oder 6-gliedrigen Ring oder stehen unabhängig voneinander für eine (C₁-C₆)-Alkylgruppe, eine (C₂-C₆)-Alkenylgruppe, eine Arylgruppe, eine Aryl-(C₁-C₆)-alkylgruppe, eine (C₂-C₆)-Hydroxyalkylgruppe, eine (C₂-C₆)-Polyhydroxyalkylgruppe oder eine Gruppe R^{I}R^{II}N-(CH₂)ₘ-, worin R^{I} und R^{II} stehen unabhängig voneinander für ein Wasserstoffatom, eine (C₁-C₆)-Alkylgruppe, eine (C₁-C₆)-Alkenylgruppe oder eine Aryl-C₁-C₆-alkylgruppe, wobei R^{I} und R^{II} gemeinsam mit dem Stickstoffatom einen 5-, 6- oder 7-gliedrigen Ring bilden können und m steht für eine Zahl 2, 3, 4, 5 oder 6, und
- R¹⁵ ein Wasserstoffatom, eine (C₁-C₆)-Alkylgruppe, eine (C₂-C₆)-Alkenylgruppe, eine Arylgruppe, eine Aryl-(C₁-C₆)-alkylgruppe, eine (C₂-C₆)-Hydroxyalkylgruppe, eine (C₂-C₆)-Polyhydroxyalkylgruppe oder eine Gruppe R^{III}R^{IV}N-(CH₂)ₙ-, worin R^{III} und R^{IV} stehen unabhängig voneinander für ein Wasserstoffatom, eine (C₁-C₆)-Alkylgruppe, eine (C₁-C₆)-Alkenylgruppe oder eine Aryl-C₁-C₆-alkylgruppe, wobei R^{III} und R^{IV} gemeinsam mit dem Stickstoffatom einen 5-, 6- oder 7-gliedrigen Ring bilden können und n steht für eine Zahl 2, 3, 4, 5 oder 6
- Y steht für ein Sauerstoffatom, ein Schwefelatom oder eine Gruppe NR^{VII}, worin R^{VII} steht für ein Wasserstoffatom, eine Arylgruppe, eine Heteroarylgruppe, eine C₁-C₆-Alkylgruppe oder eine C₁-C₆-Arylalkylgruppe,
- X- steht für ein physiologisch verträgliches Anion,
- Het steht für einen gegebenenfalls substituierten Heteroaromaten,
- X¹ steht für eine direkte Bindung oder eine Carbonylgruppe.

Gleichwirkend zu den Verbindungen der Formel IX sind deren Enaminformen. Es wird hier ausdrücklich auf die Druckschrift WO-A1-2004/022016 verwiesen, auf die vollinhaltlich Bezug genommen wird.

Mindestens eine Gruppe R¹⁰ oder R¹² gemäß Formel IX steht zwingend für eine C₁-C₆-Alkylgruppe. Diese Alkylgruppe trägt an deren alpha-Kohlenstoffatom bevorzugt mindestens zwei Wasserstoffatome. Besonders bevorzugte Alkylgruppen sind die Methyl-, Ethyl-, Propyl-, n-Butyl-, iso-Butyl, n-Pentyl-, neo-Pentyl-, n-Hexylgruppe. Ganz besonders bevorzugt stehen R¹⁰ und R¹² unabhängig voneinander für Wasserstoff oder eine Methylgruppe, wobei mindestens eine Gruppe R¹⁰ oder R¹² eine Methylgruppe bedeutet.

In einer bevorzugten Ausführungsform steht Y in Formel (IX) für ein Sauerstoff- oder ein Schwefelatom, besonders bevorzugt für ein Sauerstoffatom.

Der Rest R⁸ in Formel (IX) wird bevorzugt ausgewählt aus einer (C₁-C₆)-Alkylgruppe (besonders bevorzugt einer Methylgruppe), einer C₂-C₆-Alkenylgruppe (insbesondere einer Allylgruppe), einer Hydroxy-(C₂- bis C₆)-alkylgruppe, insbesondere einer 2-Hydroxyethylgruppe, oder einer gegebenenfalls substituierten Benzylgruppe.

R¹¹ steht bevorzugt für ein Wasserstoffatom.

Besonders bevorzugt stehen die Reste R⁹, R¹⁰ und R¹² für eine Methylgruppe, der Rest R¹¹ für ein Wasserstoffatom, Y für ein Sauerstoff- oder ein Schwefelatom und der Rest R⁸ wird ausgewählt aus einer (C₁-C₆)-Alkylgruppe (besonders bevorzugt einer Methylgruppe), einer C₂-C₆-Alkenylgruppe (insbesondere einer Allylgruppe), einer Hydroxy-(C₂- bis C₆)-alkylgruppe, insbesondere einer 2-Hydroxyethylgruppe, oder einer gegebenenfalls substituierten Benzylgruppe.

Vorzugsweise sind die Verbindungen gemäß Formel IX ausgewählt aus einer oder mehrerer Verbindungen der Gruppe von Salzen mit physiologisch verträglichem Gegenion X⁻, die gebildet wird aus Salzen des
1,2-Dihydro-1,3,4,6-tetramethyl-2-oxo-pyrimidiniums,
1,2-Dihydro-1,3-diethyl-4,6-dimethyl-2-oxo-pyrimidiniums,
1,2-Dihydro-1,3-dipropyl-4,6-dimethyl-2-oxo-pyrimidiniums,
1,2-Dihydro-1,3-di(2-hydroxyethyl)-4,6-dimethyl-2-oxo-pyrimidiniums,
1,2-Dihydro-1,3-diphenyl-4,6-dimethyl-2-oxo-pyrimidiniums,
1,2-Dihydro-1,3,4-trimethyl-2-oxo-pyrimidiniums,
1,2-Dihydro-1,3-diethyl-4-methyl-2-oxo-pyrimidiniums,
1,2-Dihydro-1,3-dipropyl-4-methyl-2-oxo-pyrimidiniums,
1,2-Dihydro-1,3-di(2-hydroxyethyl)-4-methyl-2-oxo-pyrimidiniums,
1,2-Dihydro-1,3-diphenyl-4-methyl-2-oxo-pyrimidiniums,
1 -Allyl-1,2-dihydro-3,4,6-trimethyl-2-oxo-pyrimidiniums,
1,2-Dihydro-1-(2-hydroxyethyl)-3,4,6-trimethyl-2-oxo-pyrimidiniums,
1,2-Dihydro-1,3,4,6-tetramethyl-2-thioxo-pyrimidiniums,
1,2-Dihydro-1,3-diethyl-4,6-dimethyl-2-thioxo-pyrimidiniums,
1,2-Dihydro-1,3-dipropyl-4,6-dimethyl-2-thioxo-pyrimidiniums,
1,2-Dihydro-1,3-di(2-hydroxyethyl)-4,6-dimethyl-2-thioxo-pyrimidiniums,
1,2-Dihydro-1,3-diphenyl-4,6-dimethyl-2-thioxo-pyrimidiniums,
1,2-Dihydro-1,3,4-trimethyl-2-thioxo-pyrimidiniums,
1,2-Dihydro-1,3-diethyl-4-methyl-2-thioxo-pyrimidiniums,
1,2-Dihydro-1,3-dipropyl-4-methyl-2-thioxo-pyrimidiniums,
1,2-Dihydro-1,3-di(2-hydroxyethyl)-4-methyl-2-thioxo-pyrimidiniums,
1,2-Dihydro-1,3-diphenyl-4-methyl-2-thioxo-pyrimidiniums,
1,2-Dihydro-3,4-dimethyl-2-oxo-chinazofiniums und
1,2-Dihydro-3,4-dimethyl-2-thioxo-chinazoliniums.

Ganz besonders bevorzugte erfindungsgemäße Mittel sind dadurch gekennzeichnet, dass sie als CH-acide Verbindung
Salze des 1,2-Dihydro-1,3,4,6-tetramethyl-2-oxopyrimidiniums,
Salze des 1,2-Dihydro-1,3,4-trimethyl-2-oxopyrimidiniums,
Salze des 1,2-Dihydro-1,3,4,6-tetramethyl-2-thioxopyrimidiniums,
Salze des 1-Allyl-1,2-dihydro-3,4,6-trimethyl-2-oxopyrimidiniums,
Salze des 1,2-Dihydro-1-(2-hydroxyethyl)-3,4,6-trimethyl-2-oxopyrimidiniums, 2-(Cyanomethyl)benzimidazol,
4,5-Dihydro-4-imino-2-(1-piperidinyl)-thiazol und/oder dessen Hydrochlorid,
4,5-Dihydro-4-imino-2-(4-morpholinyl)-thiazol und/oder dessen Hydrochlorid,
4,5-Dihydro-4-imino-2-(1-pyrrolidinyl)-thiazol und/oder dessen Hydrochlorid
enthalten.

X⁻ steht in Formel (IX) sowie in obigen Listen bevorzugt für Halogenid, Benzolsulfonat, p-Toluolsulfonat, C₁-C₄-Alkansulfonat, Trifluormethansulfonat, Perchlorat, 0.5 Sulfat, Hydrogensulfat, Tetrafluoroborat, Hexafluorophosphat oder Tetrachlorozinkat. Besonders bevorzugt werden die Anionen Chlorid, Bromid, Iodid, Hydrogensulfat oder p-Toluolsulfonat als X- eingesetzt.

Der Rest Het gemäß Formel (X) steht bevorzugt für das Molekülfragment mit der Formel (XII), 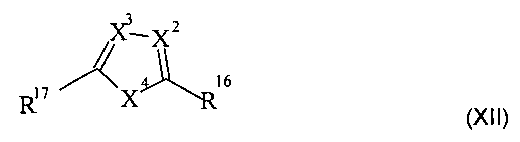 worin
- R¹⁶ und R¹⁷ stehen unabhängig voneinander für ein Wasserstoffatom, eine Hydroxygruppe, ein Halogenatom, eine Nitrogruppe, eine lineare oder zyklische C₁-C₆-Alkylgruppe, eine C₂-C₆-Alkenylgruppe, eine gegebenenfalls substituierte Arylgruppe, eine Cyanmethylgruppe, eine Cyanmethylcarbonylgruppe, eine gegebenenfalls substituierte Heteroarylguppe, eine Aryl-C₁-C₆-alkylgruppe, eine C₁-C₆-Hydroxyalkylgruppe, eine C₂-C₆-Polyhydroxyalkylgruppe, eine C₁-C₆-Alkoxygruppe, eine C₁-C₆-Alkoxycarbonylgruppe, eine C₁-C₆-Alkoxy-C₂-C₆-alkylgruppe, eine C₁-C₆-Sulfoalkylgruppe, eine C₁-C₆-Carboxyalkylgruppe, eine Gruppe R^{VIII}R^{IX}N-(CH₂)ₘ-, worin R^{VIII} und R^{IX} stehen unabhängig voneinander für ein Wasserstoffatom, eine lineare oder zyklische C₁-C₆-Alkylgruppe, eine C₂-C₆-Alkenylgruppe, eine C₁-C₆-Hydroxyalkylgruppe oder eine Aryl-C₁-C₄-alkylgruppe, wobei R^{VIII} und R^{IX} gemeinsam mit dem Stickstoffatom einen 5-, 6- oder 7-gliedrigen Ring bilden können und m steht für eine Zahl 0, 1, 2, 3 oder 4,
   wobei R¹⁶ und/oder R¹⁷ einen an den Ring des Restmoleküls ankondensierten, gegebenenfalls substituierten aromatischen oder heteroaromatischen, 5- oder 6-Ring bilden können
- X² und X³ stehen unabhängig voneinander für ein Stickstoffatom oder eine Gruppe CR¹⁵, wobei R¹⁵ steht für ein Wasserstoffatom, eine Hydroxygruppe, ein Halogenatom, eine Nitrogruppe, eine lineare oder zyklische C₁-C₆-Alkylgruppe, eine C₂-C₆-Alkenylgruppe, eine gegebenenfalls substituierte Arylgruppe, eine Cyanmethylgruppe, eine Cyanmethylcarbonylgruppe, eine gegebenenfalls substituierte Heteroarylguppe, eine Aryl-C₁-C₆-alkylgruppe, eine C₁-C₆-Hydroxyalkylgruppe, eine C₂-C₆-Polyhydroxyalkylgruppe, eine C₁-C₆-Alkoxygruppe, eine C₁-C₆-Alkoxycarbonylgruppe, eine C₁-C₆-Alkoxy-C₂-C₆-alkylgruppe, eine C₁-C₆-Sulfoalkylgruppe, eine C₁-C₆-Carboxyalkylgruppe und eine Gruppe R^{X}R^{XI}N-(CH₂)ₙ-, worin R^{X} und R^{XI} stehen unabhängig voneinander für ein Wasserstoffatom, eine lineare oder zyklische C₁-C₆-Alkylgruppe, eine C₂-C₆-Alkenylgruppe, eine C₁-C₆-Hydroxyalkylgruppe oder eine Aryl-C₁-C₄-alkylgruppe, wobei R^{X} und R^{XI} gemeinsam mit dem Stickstoffatom einen 5-, 6- oder 7-gliedrigen Ring bilden können und n steht für eine Zahl 0, 1, 2, 3 oder 4,
   wobei mindestens einer der Substituenten X² und X³ zusammen mit dem Restmolekül einen ankondensierten gegebenenfalls substituierten aromatischen 5-oder 6-Ring bilden kann,
- X⁴ steht für ein Sauerstoffatom, ein Schwefelatom, einer Vinylengruppe oder eine Gruppe N-H, wobei die beiden letztgenannten Gruppen unabhängig voneinander gegebenenfalls mit einer linearen oder zyklischen C₁-C₆-Alkylgruppe, einer C₂-C₆-Alkenylgruppe, einer gegebenenfalls substituierten Arylgruppe, einer gegebenenfalls substituierten Heteroarylguppe, einer Aryl-C₁-C₆-alkylgruppe, einer C₂-C₆-Hydroxyalkylgruppe, einer C₂-C₆-Polyhydroxyalkylgruppe, einer C₁-C₆-Alkoxy-C₂-C₆-alkylgruppe, einer C₁-C₆-Sulfoalkylgruppe, einer C₁-C₆-Carboxyalkylgruppe, einer Gruppe R^{XII}R^{XIII}N-(CH₂)ₚ- steht, worin R^{XII} und R^{XIII} stehen unabhängig voneinander für ein Wasserstoffatom, eine lineare oder zyklische C₁-C₆-Alkylgruppe, eine C₂-C₆-Alkenylgruppe, eine C₁-C₆-Hydroxyalkylgruppe oder eine Aryl-C₁-C₄-alkylgruppe, wobei R^{XII} und R^{XIII} gemeinsam mit dem Stickstoffatom einen 5-, 6- oder 7-gliedrigen Ring bilden können und p steht für eine Zahl 0, 1, 2, 3 oder 4, substituiert sein können,
mit der Maßgabe, daß, wenn X⁴ für eine Vinylengruppe steht, mindestens eine der Gruppen X² oder X³ ein Stickstoffatom bedeutet.

Die Bindung des heterozyklischen Rings gemäß Formel (XII) zum Molekülfragment -X¹-CH₂-C≡N unter Erhalt der erfindungsgemäßen Verbindung gemäß Formel (X) erfolgt an den Ring des Heterozyklusses und ersetzt ein an diesen Ring gebundenes Wasserstoffatom. Folglich ist es zwingend notwendig, daß die Substituenten R¹⁶, R¹⁷, X², X³ und X⁴ derart gewählt werden müssen, daß mindestens einer dieser Substituenten eine entsprechende Bindungsbildung ermöglicht. Folglich ist es zwingend, daß mindestens einer der Reste R¹⁶ oder R¹⁷ die Bindung zum Molekülfragment -X¹-CH₂-C≡N ausbildet, wenn X⁴ ein Sauerstoffatom oder ein Schwefelatom ist und X² und X³ ein Stickstoffatom bedeuten.

Der Rest Het gemäß Formel (X) wird besonders bevorzugt abgeleitet von den Heteroaromaten Furan, Thiophen, Pyrrol, Isoxazol, Isothiazol, Imidazol, Oxazol, Thiazol, Pyridin, Pyridazin, Pyrimidin, Pyrazin, 1,2,3-Triazin, 1,2,4-Triazin, 1,3,5-Triazin, Benzopyrrol, Benzofuran, Benzothiophen, Benzimidazol, Benzoxazol, Indazol, Benzoisoxazol, Benzoisothiazol, Indol, Chinolin, Isochinolin, Cinnolin, Phthalazin, Chinazolin, Chinoxalin, Acridin, Benzochinolin, Benzoisochinolin, Benzothiazol, Phenazin, Benzocinnolin, Benzochinazolin, Benzochinoxalin, Phenoxazin, Phenothiazin, Nephthyridin, Phenanthrolin, Indolizin, Chinolizin, Carbolin, Purin, Pteridin und Cumarin, wobei die vorgenannten Heteroaromaten mit mindestens einer Gruppe ausgewählt aus einem Halogenatom, einer Nitrogruppe, einer Thiogruppe, einer Thio-(C₁-C₆)-alkylgruppe, einer Heteroarylgruppe, einer Arylgruppe, einer (C₁-C₆)-Alkylgruppe, einer (C₁-C₆)-Alkoxygruppe, einer Hydroxygruppe, einer (C₂-C₆)-Hydroxyalkylgruppe, einer (C₂-C₆)-Polyhydroxyalkylgruppe, einer (C₁-C₆)-Alkoxyl-(C₁-C₆)-alkylgruppe, einer Aryl-(C₁-C₆)-alkylgruppe, einer Aminogruppe, einer (C₁-C₆)-Monoalkylaminogruppe, einer (C₁-C₆)-Dialkylaminogruppe, eine Dialkylaminoalkylgruppe -(CH₂)ₙ-NR'R", worin n eine ganze Zahl von 2 und 6 ist und R' und R" unabhängig voneinander eine lineare oder verzweigte Alkylgruppe bedeutet, welche gegebenenfalls zusammen einen Ring bilden können, substituiert sein können.

Vorzugsweise sind die Verbindungen gemäß Formel (X) ausgewählt aus der Gruppe bestehend aus 2-(2-Furoyl)-acetonitril, 2-(5-Brom-2-furoyl)-acetonitril, 2-(5-Methyl-2-trifluormethyl-3-furoyl)-acetonitril, 3-(2,5-Dimethyl-3-furyl)-3-oxopropanitril, 2-(2-Thenoyl)-acetonitril, 2-(3-Thenoyl)-acetonitril, 2-(5-Fluor-2-thenoyl)-acetonitril, 2-(5-Chlor-2-thenoyl)-acetonitril, 2-(5-Brom-2-thenoyl)-acetonitril, 2-(5-Methyl-2-thenoyl)-acetonitril, 2-(2,5-Dimethylpyrrol-3-oyl)-acetonitril, 2-(1,2,5-Trimethylpyrrol-3-oyl)-acetonitril, 1*H-*Benzimidazol-2-ylacetonitril, 1*H*-Benzothiazol-2-ylacetonitril, 2-(Pyrid-2-yl)-acetonitril, 2,6-Bis(cyanmethyl)-pyridin, 2-(Indol-3-oyl)-acetonitril, 2-(2-Methyl-indol-3-oyl)-acetonitril, 8-Cyanacetyl-7-methoxy-4-methylcumarin, 2-(2-Isopropyl-5,6-benzochinolin-4-oyl)-acetonitril, 2-(2-Phenyl-5,6-benzochinolin-4-oyl)-acetonitril, 2-(Chinoxalin-2-yl)-acetonitril, 2-(Cumaron-2-yl)-acetonitril, 6,7-Dichlor-5-(cyanoacetyl)-2,3-dihydro-1-benzofuran-2-carbonsäure-tert.-butylester, 2-(6-Hydroxy-4,7-dimethoxy-1-benzofuran-5-oyl)-acetonitril und 2-(1-Phenyl-1,4-dihydrothiochromeno[4,3-c]pyrazol-3-oyl)-acetonitril. Besonders bevorzugt ist 1*H*-Benzimidazol-2-ylacetonitril [2-(Cyanomethyl)benzimidazol].

Erfindungsgemäß bevorzugt im erfindungsgemäßen Verfahren eingesetzte Anwendungsmischungen sind dadurch gekennzeichnet, daß sie als Haarfärbemittel formuliert sind, die bezogen auf ihr Gewicht 0,01 bis 20 Gew.-%, vorzugsweise 0,05 bis 15 Gew.-%, besonders bevorzugt 0,1 bis 12,5 Gew.-% und insbesondere 0,2 bis 10 Gew.-% einer oder mehrerer CH-acider Verbindung(en) enthalten.

Ein weiterer Gegenstand der vorliegenden Erfindung ist die Verwendung von Zubereitungen, die mindestens ein wasserlösliches Proteinhydrolysat oder dessen wasserlösliche Alkali- und/oder (gegebenenfalls substituierte) Ammonium- und/oder Erdalkalisalze enthalten, zur Verbesserung der inneren Haarstruktur. Besonders bevorzugte erfindungsgemäße Verwendungen sind dadurch gekennzeichnet, daß die Zubereitungen, die mindestens ein wasserlösliches Proteinhydrolysat oder dessen wasserlösliche Alkali- und/oder (gegebenenfalls substituierte) Ammonium- und/oder Erdalkalisalze enthalten, in Kombination mit Zubereitungen, die wasserlösliche Calcium- und/oder Aluminiumsalze enthalten, angewendet werden.

Ein weiterer Gegenstand der vorliegenden Erfindung ist die Verwendung von Calcium- und/oder Aluminiumcaseinaten zur Verbesserung der inneren Haarstruktur.

Bezüglich bevorzugter Ausführungsformen der erfindungsgemäßen Verwendungen gilt mutatis mutandis das zu den erfindungsgemäßen Verfahren Gesagte.

## Patentansprüche

1. Verfahren zur Blondierung keratinischer Fasern, insbesondere menschlicher Haare, bei dem
- in einem ersten Schritt
o eine Zubereitung A, enthaltend mindestens ein Oxidationsmittel, ausgewählt aus Wasserstoffperoxid und dessen Anlagerungsverbindungen an feste Träger,
o gegebenenfalls mit einer Zubereitung B, enthaltend mindestens einen Bleichkraftverstärker,
o gegebenenfalls mit einer Zubereitung C zu einer Anwendungszubereitung vermischt wird,
- diese in einem zweiten Schritt auf die keratinischen Fasern aufgetragen wird und
- anschließend nach einer Einwirkzeit t₁ von den Fasern abgespült wird,
**dadurch gekennzeichnet, dass** mindestens eine der Zubereitungen A, B oder C mindestens ein wasserlösliches Proteinhydrolysat oder dessen wasserlösliche Alkali- und/oder (gegebenenfalls substituierte) Ammonium- und/oder Erdalkalisalze enthält, so daß die Anwendungszubereitung mindestens ein wasserlösliches Proteinhydrolysat oder dessen wasserlösliche Alkali- und/oder (gegebenenfalls substituierte) Ammonium- und/oder Erdalkalisalze enthält.

2. Verfahren nach Anspruch 1, **dadurch gekennzeichnet, daß**
- in einem dritten Schritt eine wäßrige Zubereitung D eines oder mehrerer wasserlöslicher Salze von Calcium und/oder Aluminium und/oder Zink und/oder der Seltenerdmetalle auf die keratinischen Fasern aufgetragen wird und diese
- anschließend nach einer Einwirkzeit t₂ von den Fasern abgespült wird.

3. Verfahren nach einem der Ansprüche 1 oder 2, **dadurch gekennzeichnet, daß** die Zubereitung A wässrig ist.

4. Verfahren nach einem der Ansprüche 1 bis 3, **dadurch gekennzeichnet, daß** die Zubereitung B wasserfrei formuliert ist, wobei bevorzugte Zubereitungen B pulverförmig sind.

5. Verfahren nach einem der Ansprüche 1 bis 4, **dadurch gekennzeichnet, daß** mindestens eine der Zubereitungen A und/oder B und/oder C mindestens ein wasserlösliches Proteinhydrolysat mit Molmassen von 400 bis 10.000 Dalton, vorzugsweise von 1000 bis 5000 Dalton oder dessen wasserlösliche Alkali- oder Erdalkalisalze enthält.

6. Verfahren nach Anspruch 5, **dadurch gekennzeichnet, daß** sämtliche in den Zubereitungen A und/oder B und/oder C enthaltenen wasserlöslichen Proteinhydrolysate und/oder deren wasserlösliche Alkali- und/oder (gegebenenfalls substituierte) Ammonium- und/oder Erdalkalisalze Molmassen von 400 bis 10.000 Dalton, vorzugsweise von 1000 bis 5000 Dalton aufweisen.

7. Verfahren nach einem der Ansprüche 1 bis 6, **dadurch gekennzeichnet, daß** das bzw. die wasserlösliche(n) Proteinhydrolysat(e) oder dessen bzw deren wasserlösliche(s) Alkali- und/oder (gegebenenfalls substituierte) Ammonium- und/oder Erdalkalisalz(e) ausgewählt ist aus Caseinhydrolysaten.

8. Verfahren nach einem der Ansprüche 1 bis 7, **dadurch gekennzeichnet, daß** das bzw. die wasserlösliche(n) Proteinhydrolysat(e) oder dessen bzw deren wasserlösliche(s) Alkali- und/oder (gegebenenfalls substituierte) Ammonium- und/oder Erdalkalisalz(e) Phenylalanin, vorzugsweise in Mengen von 2 bis 10 g/100 g Protein, besonders bevorzugt von 3 bis 7 g/100 g Protein und insbesondere von 4 bis 6 g/100 g Protein enthalten.

9. Verfahren nach einem der Ansprüche 1 bis 8, **dadurch gekennzeichnet, daß** das bzw. die wasserlösliche(n) Proteinhydrolysat(e) oder dessen bzw. deren wasserlösliche(s) Alkali- und/oder (gegebenenfalls substituierte) Ammonium- und/oder Erdalkalisalz(e) Leucin, vorzugsweise in Mengen von 5 bis 15 g/100 g Protein, besonders bevorzugt von 6,5 bis 12 g/100 g Protein und insbesondere von 7,5 bis 10 g/100 g Protein enthalten.

10. Verfahren nach einem der Ansprüche 1 bis 9, **dadurch gekennzeichnet, daß** das bzw. die wasserlösliche(n) Proteinhydrolysat(e) oder dessen bzw deren wasserlösliche(s) Alkali- und/oder (gegebenenfalls substituierte) Ammonium- und/oder Erdalkalisalz(e) Isoleucin, vorzugsweise in Mengen von 2 bis 10 g/100 g Protein, besonders bevorzugt von 3 bis 7 g/100 g Protein und insbesondere von 4 bis 6 g/100 g Protein enthalten.

11. Verfahren nach einem der Ansprüche 1 bis 10, **dadurch gekennzeichnet, daß** das bzw. die wasserlösliche(n) Proteinhydrolysat(e) oder dessen bzw. deren wasserlösliche(s) Alkali- und/oder (gegebenenfalls substituierte) Ammonium- und/oder Erdalkalisalz(e) Threonin, vorzugsweise in Mengen von 2 bis 10 g/100 g Protein, besonders bevorzugt von 2,5 bis 7 g/100 g Protein und insbesondere von 3 bis 5,5 g/100 g Protein enthalten.

12. Verfahren nach einem der Ansprüche 1 bis 11, **dadurch gekennzeichnet, daß** das bzw. die wasserlösliche(n) Proteinhydrolysat(e) oder dessen bzw deren wasserlösliche(s) Alkali- und/oder (gegebenenfalls substituierte) Ammonium- und/oder Erdalkalisalz(e) Methionin, vorzugsweise in Mengen von 1 bis 10 g/100 g Protein, besonders bevorzugt von 1,5 bis 5 g/100 g Protein und insbesondere von 2 bis 4 g/100 g Protein enthalten.

13. Verfahren nach einem der Ansprüche 1 bis 12, **dadurch gekennzeichnet, daß** das bzw. die wasserlösliche(n) Proteinhydrolysat(e) oder dessen bzw. deren wasserlösliche(s) Alkali- und/oder (gegebenenfalls substituierte) Ammonium- und/oder Erdalkalisalz(e) Lysin, vorzugsweise in Mengen von 2 bis 12 g/100 g Protein, besonders bevorzugt von 4 bis 9 g/100 g Protein und insbesondere von 6 bis 8 g/100 g Protein enthalten.

14. Verfahren nach einem der Ansprüche 1 bis 13, **dadurch gekennzeichnet, daß** das bzw. die wasserlösliche(n) Proteinhydrolysat(e) oder dessen bzw. deren wasserlösliche(s) Alkali- und/oder (gegebenenfalls substituierte) Ammonium- und/oder Erdalkalisalz(e) Valin, vorzugsweise in Mengen von 2 bis 10 g/100 g Protein, besonders bevorzugt von 3 bis 7,5 g/100 g Protein und insbesondere von 5 bis 6,5 g/100 g Protein enthalten.

15. Verfahren nach einem der Ansprüche 1 bis 14, **dadurch gekennzeichnet, daß** das bzw. die wasserlösliche(n) Proteinhydrolysat(e) oder dessen bzw. deren wasserlösliche(s) Alkali- und/oder (gegebenenfalls substituierte) Ammonium- und/oder Erdalkalisalz(e) Histidin, vorzugsweise in Mengen von 1,2 bis 10 g/100 g Protein, besonders bevorzugt von 1,7 bis 5 g/100 g Protein und insbesondere von 2 bis 4 g/100 g Protein enthalten.

16. Verfahren nach einem der Ansprüche 1 bis 15, **dadurch gekennzeichnet, daß** das bzw. die wasserlösliche(n) Proteinhydrolysat(e) oder dessen bzw. deren wasserlösliche(s) Alkali- und/oder (gegebenenfalls substituierte) Ammonium- und/oder Erdalkalisalz(e) Arginin, vorzugsweise in Mengen von 2 bis 10 g/100 g Protein, besonders bevorzugt von 2,3 bis 7 g/100 g Protein und insbesondere von 3 bis 6 g/100 g Protein enthalten.

17. Verfahren nach einem der Ansprüche 1 bis 16, **dadurch gekennzeichnet, daß** das bzw. die wasserlösliche(n) Proteinhydrolysat(e) oder dessen bzw. deren wasserlösliche(s) Alkali- und/oder (gegebenenfalls substituierte) Ammonium- und/oder Erdalkalisalz(e) Prolin, vorzugsweise in Mengen von 5 bis 15 g/100 g Protein, besonders bevorzugt von 6 bis 12 g/100 g Protein und insbesondere von 8 bis 10,5 g/100 g Protein enthalten.

18. Verfahren nach einem der Ansprüche 1 bis 17, **dadurch gekennzeichnet, daß** das bzw. die wasserlösliche(n) Proteinhydrolysat(e) oder dessen bzw. deren wasserlösliche(s) Alkali- und/oder (gegebenenfalls substituierte) Ammonium- und/oder Erdalkalisalz(e) Alanin, vorzugsweise in Mengen von 1,5 bis 10 g/100 g Protein, besonders bevorzugt von 2 bis 7 g/100 g Protein und insbesondere von 2,5 bis 4 g/100 g Protein enthalten.

19. Verfahren nach einem der Ansprüche 1 bis 18, **dadurch gekennzeichnet, daß** das bzw. die wasserlösliche(n) Proteinhydrolysat(e) oder dessen bzw. deren wasserlösliche(s) Alkali- und/oder (gegebenenfalls substituierte) Ammonium- und/oder Erdalkalisalz(e) Asparaginsäure, vorzugsweise in Mengen von 2 bis 10 g/100 g Protein, besonders bevorzugt von 3 bis 8 g/100 g Protein und insbesondere von 5 bis 7 g/100 g Protein enthalten.

20. Verfahren nach einem der Ansprüche 1 bis 19, **dadurch gekennzeichnet, daß** das bzw. die wasserlösliche(n) Proteinhydrolysat(e) oder dessen bzw. deren wasserlösliche(s) Alkali- und/oder (gegebenenfalls substituierte) Ammonium- und/oder Erdalkalisalz(e) Serin, vorzugsweise in Mengen von 2 bis 10 g/100 g Protein, besonders bevorzugt von 3 bis 7 g/100 g Protein und insbesondere von 4 bis 6 g/100 g Protein enthalten.

21. Verfahren nach einem der Ansprüche 1 bis 20, **dadurch gekennzeichnet, daß** das bzw. die wasserlösliche(n) Proteinhydrolysat(e) oder dessen bzw. deren wasserlösliche(s) Alkali- und/oder (gegebenenfalls substituierte) Ammonium- und/oder Erdalkalisalz(e) Glutaminsäure, vorzugsweise in Mengen von 10 bis 30 g/100 g Protein, besonders bevorzugt von 15 bis 25 g/100 g Protein und insbesondere von 18 bis 22 g/100 g Protein enthalten.

22. Verfahren nach einem der Ansprüche 1 bis 21, **dadurch gekennzeichnet, daß** das bzw. die wasserlösliche(n) Proteinhydrolysat(e) oder dessen bzw. deren wasserlösliche(s) Alkali- und/oder (gegebenenfalls substituierte) Ammonium- und/oder Erdalkalisalz(e) Glycin, vorzugsweise in Mengen von 1 bis 5 g/100 g Protein, besonders bevorzugt von 1,3 bis 3 g/100 g Protein und insbesondere von 1,5 bis 2,5 g/100 g Protein enthalten.

23. Verfahren nach einem der Ansprüche 1 bis 22, **dadurch gekennzeichnet, daß** das bzw. die wasserlösliche(n) Proteinhydrolysat(e) oder dessen bzw. deren wasserlösliche(s) Alkali- und/oder (gegebenenfalls substituierte) Ammonium- und/oder Erdalkalisalz(e) Tyrosin, vorzugsweise in Mengen von 2 bis 10 g/100 g Protein, besonders bevorzugt von 3 bis 7 g/100 g Protein und insbesondere von 4 bis 6 g/100 g Protein enthalten.

24. Verfahren nach einem der Ansprüche 2 bis 23, **dadurch gekennzeichnet, daß** die Zubereitung D bezogen auf ihr Gewicht 2,5 bis 30 Gew.-%, vorzugsweise 5 bis 20 Gew.-% und insbesondere 7,5 bis 15 Gew.-% Calcium- und/oder Aluminiumsalze enthält, wobei bevorzugte Salze ausgewählt sind aus CaCl₂, Al₂(SO₄)₃, NaAl(SO₄)₂, KAI(SO₄)₂, Aluminiumlactat und deren Mischungen.

25. Verwendung von Zubereitungen, die mindestens ein wasserlösliches Proteinhydrolysat oder dessen wasserlösliche Alkali- und/oder (gegebenenfalls substituierte) Ammonium- und/oder Erdalkalisalze enthalten, zur Verbesserung der inneren Haarstruktur.

26. Verwendung nach Anspruch 25, **dadurch gekennzeichnet, daß** die Zubereitungen, die mindestens ein wasserlösliches Proteinhydrolysat oder dessen wasserlösliche Alkali- und/oder (gegebenenfalls substituierte) Ammonium- und/oder Erdalkalisalze enthalten, in Kombination mit Zubereitungen, die wasserlösliche Calcium- und/oder Aluminiumsalze enthalten, angewendet werden.

27. Verwendung von Calcium- und/oder Aluminiumcaseinaten zur Verbesserung der inneren Haarstruktur.
